# EUROPEAN PATENT SPECIFICATION

(11) **EP 4 074 843 B1**
(45) Date of publication and mention of the grant of the patent: **11.03.2026**
(21) Application number: 20897930.2
(22) Date of filing: 11.12.2020
(51) Int. Cl.: C12Q 1/70, C12Q 1/68, C12Q 1/6844, C12Q 1/6888, C12N 15/11, C12R 1/93

(54) **METHOD AND TEST KIT FOR DETECTING HUMAN PAPILLOMAVIRUS**
VERFAHREN UND TESTKIT ZUM NACHWEIS VON MENSCHLICHEM PAPILLOMAVIRUS
PROCÉDÉ ET KIT D'ESSAI POUR LA DÉTECTION DE PAPILLOMAVIRUS HUMAIN

(30) Priority: 13.12.2019 CN 201911283675
(43) Date of publication of application: 19.10.2022
(73) Proprietor: Zhejiang Wolwo Bio-pharmaceutical Co., Ltd., Zhejiang 313200 (CN)
(72) Inventor: HU, Gengxi, Shanghai 200233 (CN); GUAN, Zhenwei, Shanghai 200233 (CN)
(74) Representative: Pace Napoleone, Maria
(86) International application number: PCT/CN2020/135565
(87) International publication number: WO 2021/115406

(56) References cited:
- WO-A1-2016/005789
- WO-A1-2017/001544
- WO-A1-2018/235109
- WO-A2-2011/103159
- CN-A- 101 851 630
- CN-A- 104 293 974
- CN-A- 104 560 962
- CN-A- 104 630 384
- CN-A- 105 603 121
- CN-A- 107 119 147

## Description

### TECHNICAL FIELD

The present invention pertains to the field of biotechnology, and in particular, relates to a method and a kit for detecting human papillomaviruses.

### BACKGROUND

Cervical cancer ranks the second most common female malignancy worldwide, with around 530,000 new cases and 274000 deaths being reported annually. It can take quite long for cervical cancer to develop and to progress. Typically, it may take over 10 to 20 years for precancerosis to develop into invasive carcinoma through carcinoma in situ. Given the long reversible precancerosis phase before onset, early diagnosis at the precancerosis stage, which begets follow-up treatment or testing, is believed crucial to reduce incidence and mortality of cervical cancer.

Human Papillomavirus (HPV), belonging to the family Papillomaviridae, is a small, non-enveloped circular double-stranded DNA virus with a genome consisting of approximately 8000 base pairs (bp). The genome is divided into 3 functional regions: the early coding region (E region), the late coding region (L region) and the non-coding region (long control region, LCR). HPV infection is a major cause of cervical intraepithelial neoplasia and cervical cancer. The results of studies worldwide showed that presence of high-risk HPV DNAs was detected in 99.7% of patients with cervical cancer. Currently, formal classification of HPV is based on sequence typing of the L1 region (Ethel-Michele de Villiers, Claude Fauquet, Thomas R Broker. Classification of papillomaviruses[J]. Virology, 2004: 324(1): 17-27.). As defined, two HPV isolates are to be classified into different types if there exists between them more than 10% sequence variation in the L1 region. According to the data from the International Human Papillomavirus (HPV) Reference Center, 222 HPV genotypes have been identified, wherein around 40 are associated with human genital skin and mucous membrane lesions. The *Technical Review Guidelines for Human Papillomavirus (HPV) Nucleic Acid Detection, Genotyping and Reagents* issued by the National Medical Products Administration (NMPA), based on the research results of International Agency for Research on Cancer (IARC) of WHO and other international organizations, have suggests that 13 HPV genotypes, i.e., HPV 16, 18, 31, 33, 35, 39, 45, 51, 52, 56, 58, 59 and 68, are classified as high-risk genotypes, and 5 genotypes, i.e., HPV 26, 53, 66, 73 and 82, are classified as medium-risk genotypes. The *Guidelines* also suggests that all these 18 HPV genotypes should be assayed in clinical practice to support an early diagnosis of cervical cancer. In a further aspect, virus screening also plays an important role in quality and especially safety controls for biological products such as cell products and blood products. It is a problem to be solved that there lacks comprehensive detection of HPV viruses of medium and high risk genotypes to allow for improved the detection rate and prevention of miss-detection.

### SUMMARY OF THE INVENTION

The present invention is defined by the appended claims.

The inventors conducted a phylogenetic analysis (MegAlign software (https://www.dnastar.coml)) on the DNA sequences of the L1 region of the already known 222 HPV genotypes, and found that the above-said 18 HPV genotypes are not adjacent to one another in the phylogenetic tree. In a further study, the inventors traced to the large branch covering the above 18 HPV genotypes and identified the specific genotypes under this large branch, whereby anexpanded group of genotypes were obtained, which enables comprehensive detection of medium and high risk genotypes of the HPV virus to thereby solve the problem in the art.

In one aspect, the present invention provides a method for detecting presence of human papillomavirus (HPV) in a sample, comprising the step of detecting presence of HPVs of at least the following 65 HPV genotypes: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73 and HPV177.

In some embodiments, the presence of said genotypes is detected by polymerase chain reaction (PCR) assays, and the presence of HPV in the sample is determined according to results of said PCR assays. In some embodiments, the PCR assays are conducted using a set of primer pairs capable of detecting the at least 65 human papillomavirus genotypes, wherein one or more primer pairs is(are) degenerate primer pair(s).

In some embodiments, the method includes the following steps:
1) Obtaining DNAs from the sample;
2) Reacting the DNAs obtained in step 1) with each primer pair in said set of primer pairs capable of detecting the at least 65 human papillomavirus genotypes in a PCR assay separately, wherein, preferably, one or more primer pairs in the set is(are) degenerate primer pair(s); and
3) Determining the presence of HPV in the sample according to results of the PCR assays.

In some embodiments, the step of determining the presence of HPV in the sample according to results of the PCR assays comprises comparing the measured Ct (Threshold Cycle) in each of the PCR assays with a reference Ct; wherein the result of the PCR assay is to be determined positive when said measured Ct is less than said reference Ct or when a unimodal dissociation curve of PCR product is observed and said measured Ct is less than said reference Ct, and the result of the PCR assay is to be determined negative when said measured Ct is greater than or equal to said reference Ct or no logarithmic amplification curve is observed; and whereby, said sample is to be determined positive for presence of HPV when the PCR assay with any one of said primer pairs gives a positive result; wherein, preferably, the reference Ct ranges from 30 to 35, from 32 to 35 or from 33 to 35 or is 34 or 35.

In some embodiments, the primer pair set comprises one or more primer pairs as set forth in Table 1, wherein F and R respectively refer to the forward primer and the reverse primer as paired.

**Table 1:**

| Sequence | SEQ ID NO | Sequence | SEQ ID NO |
|---|---|---|---|
| F1: CWGGWRCWGAYAATAGRGAAAATG | 1 | R1: YRCCATATCMCCATCCTGWATA | 2 |
| F2: GCRACAAWCTTGAGGACCARAT | 3 | R2: YGTGTGCTAGATACRGGTTCAAATG | 4 |
| F3: TGCGTGCCAGGAGABGYTAATAGAC | 5 | R3: RKGTCCTGGGTRCTAGATAC | 6 |
| F4: CCYGAKGTGRTTGACCTACA | 7 | R4: VVHRTYRGCCATGTCTCTTATG | 8 |
| F5: TGCWGAGCCBTATGGCGATTG | 9 | R5: ACMCCAAAATTCCACTCATC | 10 |
| F6: TGTGGCCTAAACGACGTAAAC | 11 | R6: CGCGTGACATAGACATCCGTACTG | 12 |
| F7: GGAGAMGTTRCTAGAACTGTATGA | 13 | R7: GGYDCTGYGTCCCACATTTC | 14 |
| F8: GTGCCAGGAGAAAATACTAGACT | 15 | R8: YSCYGBAGGTACTAGATACA | 16 |
| F9: YGCCTATGGBGATTCTATGTG | 17 | R9: CCACTAGGHGTAGCAACATATAC | 18 |
| F10: GGCMCAGGGYCATAACAATGGT | 19 | R10: DCGWCCAAGRGGATATTGAT | 20 |
| F11: GCWGATGTRTATGGRGACAGTATGT | 21 | R11: RGGCTTRTTAAAYAACTGGGARTC | 22 |
| F12: TGTCCACCAATGCTTATTACA | 23 | R12: CCTCTTCCTSGTKCAAATCTAATC | 24 |
| F13: GATGGWAACCCAACTAGYATAG | 25 | R13: CCGTTGCTGTCAAATGGAAATG | 26 |
| F14: ASYGAAGTATCCTCTGTTGCGTCTA | 27 | R14: TCGAATCGCACTCGCTTTCTA | 28 |
| F15: GTTGTWAGCACKGATGAATATG | 29 | R15: CCACTAATRCCYACACCYAATG | 30 |
| F16: GACGTGARCARATGTTTGTTAG | 31 | R16: GGWGGKGTWARRCCAAATTG | 32 |
| F17: TRCACGYARACGCCGTAAAC | 33 | R17: KKCCYACWGCAAGTAGTCTA | 34 |
| F18: GGCMWTRTTAGATGATGCWACA | 35 | R18: CACGTTCATACAGTTCTAGGA | 36 |

wherein R represents A or G; Y represents C or T; M represents A or C; K represents G or T; S represents C or G; W represents A or T; H represents A, C or T; B represents C, G or T; V represents A, C or G; and D represents A, G or T.

In some embodiments, annealing temperatures for the PCR assays are independently 40°C-60°C, preferably 50°C-60°C, and more preferably 55°C.

In some embodiments, said PCR assays each comprise independently 30 to 50 cycles, preferably 35-45 cycles and more preferably 40 cycles of PCR reaction.

The method of the present invention is carried out *in vitro* or *ex vivo.* In the present invention, a sample refers to an isolated *ex vivo* sample. In some embodiments, the sample is selected from the group consisting of cells, blood or secretions. In some embodiments, the sample is a sample of cells, such as stem cells, and particularly mesenchymal stem cells (MSCs).

In a further aspect, the present invention provides a kit for detection of human papillomavirus. The kit comprises one or more reagents that is(are) capable of detecting the at least the 65 HPV genotypes as defined above.

Said reagents include or consist of a set of primer pairs capable of detecting the at least 65 HPV genotypes. Preferably, one or more primer pairs in the set is(are) degenerate primer pair(s). And, preferably, the primer pair set includes one or more primer pairs selected from the list of Table 1.

In an embodiment, the kit is one for PCR-based assays. For example, in addition to the above primers, the kit may further include other components of a polymerase chain reaction system, references and/or quality controls, etc.. The kit may further include an instruction on how to use the kit to detect the at least 65 HPV genotypes described above or indication of access to the instruction.

In some embodiments, the kit is one for assays on a sample selected from the group consisting of cells, blood or secretions. For example, in some embodiments, the sample is a sample of cells, for example, stem cells, and particularly MSCs.

In a further aspect, the present invention provides a set of primer pairs capable of detecting at least the 65 HPV genotypes as defined above for use in detection of HPV, including one or more primer pairs selected from Table 1. Provided is a set of primer pairs capable of detecting at least the 65 HPV genotypes as defined above, including one or more primer pairs selected from Table 1. For example, the set may include or consist of all the primer pairs listed in Table 1.

In a further aspect, the present invention provides use of the at least 65 HPV genotypes as defined above and/or one or more reagents capable of detecting same for manufacturing tools for detecting HPV. The tools may include software products and hardware products, such as kits, instruments, devices, systems, etc. In an embodiment, the reagents include or consist of a set of primer pairs capable of detecting the at least 65 HPV genotypes, wherein, preferably, one or more primer pairs in the set is(are) degenerate primer pair(s); and preferably, the set of primer pairs comprise one or more primer pairs selected from Table 1.

Also contemplated is a system for determining presence of HPV in a sample, wherein the system may include a device for detecting HPVs of the at least 65 HPV genotypes as defined above. The device may include hardware, software such as a program, or both, for implementing said use. In some embodiments, the system is used for detecting medium- and high-risk HPV genotypes, for disease screening, such as cervical cancer screening.

In some embodiments, the system of the invention includes a device for detecting presence of said genotypes in a sample by PCR assays, and a device for determining presence of HPV in a sample according to results of the PCR assays. Preferably, the PCR assays are conducted using a set of primer pairs capable of detecting HPVs of at least the said 65genotypes, wherein one or more primer pairs in the set is(are) degenerate primer pair(s).

In some embodiments, the system of the invention includes: 1) a device for obtaining DNAs from a sample; 2) a device for reacting the obtained DNAs with each primer pair in said set of primer pairs capable of detecting HPVs of said at least 65 genotypes in a PCR assay separately; and 3) a device for determining the presence of HPV in a sample according to results of the PCR assays. Preferably, one or more primer pairs in the set is(are) degenerate primer pair(s).

Also contemplated is a computer-readable storage medium with instructions or a program stored thereon, wherein the instructions or the program, when being executed by a processor, direct(s) performing of a process of detecting presence of HPVs of said at least 65 genotypes in a sample.

In some embodiments, the instructions or the program on the storage medium, when being executed by a processor, direct(s) performing of the following steps: detecting presence of the at least 65 HPV genotypes in a sample via PCR assays; and determining presence of HPV in a sample according to results of the PCR assays. Preferably, the PCR assays are conducted using a set of primer pairs capable of detecting the at least 65 HPV genotypes, wherein one or more primer pairs in the set is(are) degenerate primer pair(s).

In some embodiments, the instruction or the program on the storage medium, when being executed by a processor, direct(s) performing of the following steps: 1) obtaining DNAs from a sample; 2) reacting the DNAs obtained in step 1) with each primer pair in a set of primer pairs capable of detecting HPVs of said at least 65 genotypes in a PCR assay separately; and 3) determining presence of HPV in said sample according to results of said PCR assays. Preferably, one or more primer pairs in the set is(are) degenerate primer pair(s).

The storage medium may be any available medium that can be accessed by a computer. For example, useful computer-readable media may include random access memories (RAMs), read-only memories (ROMs), electrically erasable programmable read-only memories (EEPROMs), CD-ROMs or other optical-disk storage, magnetic-disk storage or other magnetic storage devices, or any other media that carry or store program codes as desired in the form of instructions or data structures and can be accessed by a computer.

The 65 genotypes according to the present invention cover the 13 high-risk genotypes and the 5 medium-risk genotypes as recommended by the International Agency for Research on Cancer (IARC) of WHO. And, all of the 65 genotypes stemmed from the same branch of the phylogenetic tree (Examples 1-4), which indicates that the 65 genotypes are closely related in phylogenesis and thus also pose risks of infection leading to development of cervical cancer.

The method and the tools according to the present invention, by encompassing the at least 65 genotypes as defined above, provide maximized detection of HPV genotypes of medium and high risks and improved efficiency in disease screening. In another aspect, the method and the tools according to the present invention, by enabling comprehensive inspection of HPVs of medium- and high-risk genotypes in samples, avoid miss-detection to the maximum and thereby provide improved product quality, safety and quality control efficiency for biological products such as cell products and blood products.

### BRIEF DESCRIPTION OF THE DRAWINGS

FIG. 1 shows a phylogenetic tree diagram for DNA sequence alignment in L1 regions of different HPV genotypes according to Example 1 and a zoom-in of selected portion.
FIG. 2 shows a phylogenetic tree diagram for DNA sequence alignment in L2 regions of different HPV genotypes according to Example 2 and a zoom-in of selected portion.
FIG. 3 shows a phylogenetic tree diagram for DNA sequence alignment in E1 regions of different HPV genotypes according to Example 3 and a zoom-in of selected portion.
FIG. 4 shows a phylogenetic tree diagram for DNA sequence alignment in E6 regions of different HPV genotypes according to Example 4 and a zoom-in of selected portion.

### DETAILED DESCRIPTION OF EMBODIMENTS OF THE INVENTION

In the present disclosure, unless otherwise specified, the involved components or the preferred components thereof can be combined with each other to form a new technical solution.

In the present disclosure, unless otherwise specified, all the embodiments and preferred embodiments mentioned herein can be combined with each other to form a new technical solution.

In the present disclosure, unless otherwise specified, all the technical features and preferred features mentioned herein can be combined with each other to form a new technical solution.

As used herein, unless otherwise specified, the terms "a" and "an" mean "at least one".

In the present disclosure, a "range" can be defined by a lower limit or an upper limit or both, wherein either the lower limit or the upper limit or both can be assigned with multiple values. For example, a range can be defined by a selected lower limit and a selected upper limit, whereby specific boundaries of the particular range are defined. All ranges that can be defined in this way are inclusive and combinable. That is, any lower limit can be combined with any upper limit to form a range without departing from the scope of the present description and claims. For example, disclosure of a range of 30-50 and a range of 35-45 for a particular parameter should be interpreted as also disclosing the ranges of 30-45 and 35-50 for the parameter.

"Polymerase chain reaction" is also referred to as "PCR" herein.

Specifically, the present invention relates to a detection method for detecting HPV genotypes and uses thereof in the field of biotechnology. The inventors found in a genetic relationship analysis (i.e., phylogenetic tree analysis) of the DNA sequences of the already known HPV genotypes a group of additional HPV genotypes that are closely related to the 18 medium- and high-risk HPV genotypes recommended in the "Guidelines for Technical Review of Nucleic Acid Detection, Genotyping and Reagents for Human Papillomavirus (HPV)" issued by China Food and Drug Administration (CFDA), including the following 65 genotypes of human papillomavirus: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73, and HPV177.

The full-length sequences and partition sequences of the HPV genotypes involved in the present invention are available from the DNA database (GenBank: https://www.ncbi.nlm.nih.gov/nuccorel) established by National Center for Biotechnology Information (NCBI), the Japan DNA Database (DDBJ: https://www.ddbi.nie.ac.jp), the European DNA Database (EMBI: https://www.embl.org), and the Master Database of Human Papillomaviruses (Human Reference clones - hpvcenter: https://www.hpvcenter.se/), and can also be obtained by other methods as well known to those skilled in the art, like gene sequencing. The genetic relationship analysis was conducted using well-established methods, such as the phylogenetic tree analysis. Multiple sequence alignment analysis was conducted using softwares including MegAlign, Cluster X, Muscle and Phylip. In a specific example, MegAlign analysis software was used. The selected tree-building methods include distance-based methods, character-based methods, etc., with distance-based methods being preferred.

The method of detection according to the present invention may be based on a polymerase chain reaction (PCR) including the specific steps of DNA denaturation, annealing, extension and cycling, or any other methods of detection known to those skilled in the art.

In some embodiments, the method may include the following steps:
1) Obtaining DNAs from the sample;
   Specifically, this may include, for example, the following steps: preparing or isolating cells from the sample; preparing the cells into a suspension in PBS at a concentration of, for example, 1×10⁵-1×10⁷ cells/ml; and obtaining a DNA extract via lysis of the cells, wherein the method of lysis may be any of those known in the art, such as mechanical lysis, chemical lysis, enzymatic lysis , or any combination thereof;
2) Reacting the DNA extract obtained in step 1) with primer pairs capable of detecting HPVs of said at least 65 genotypes in PCR assays separately;
   Generally, one PCR system contains only one primer pair including a forward (i.e., upstream) primer and a reverse (i.e., downstream) primer. As well understood, a polymerase chain reaction system may further comprise, for example, dNTPs, DNA polymerase, buffer, etc., in a volume of, for example, 20-50 µL;
3) Determining presence of HPV in the sample according to results of these PCR assays.

In another embodiment, the method may include the following steps:
1) Obtaining DNAs from the sample;
   Specifically, this may include, for example, the following steps: thoroughly eluting the secretion from a cervical sampling brush; collecting and pooling fractions of eluate, then centrifuging, for example, at 13,000 rpm for 10 minutes; discarding the supernatant to obtain the precipitate at the bottom of the tube; obtaining a DNA extract via lysis of the precipitate, wherein the method of lysis may be any of those known in the art, such as mechanical lysis, chemical lysis, enzymatic lysis, or any combination thereof;
2) Reacting the DNA extract obtained in step 1) with primer pairs capable of detecting HPVs of said at least 65 genotypes in PCR assays separately;
   One PCR system contains only one primer pair. For example, in concurrent detections using multiple primer pairs, a plurality of PCR systems may run independently in parallel. The specific steps may include, for example, mixing the primers, dNTPs, DNA polymerase, the DNA extract, a reaction buffer system and double-distilled water into a PCR system with a total volume of, for example, 20-50 µL; and optionally, film sealing the plate, centrifuging and transferring it to the amplification and detection zoon;
3) Determining presence of HPV in the sample according to results of these PCR assays.

In some embodiments, useful DNA polymerase may include Taq DNA polymerase, Super Taq DNA polymerase, UlltraPF^{™} DNA polymerase, LA Taq DNA polymerase and Tth DNA polymerase, and preferably, Taq DNA polymerase. In some embodiments, the reaction buffer system may comprise potassium chloride (KCl), magnesium chloride (MgCl₂), ammonium sulfate ((NH₄)₂SO₄) and Tris-HCl.

For the polymerase chain reaction (PCR), the annealing temperature may preferably be 40°C-60°C, more preferably 50°C-60°C, and most preferably 55°C; and the number of cycles may be 30-50, preferably 35-45, and more preferably 40.

In some embodiments, determining the presence of HPV in a sample according to results of said PCR assays may comprise comparing the measured Ct value in each of the polymerase chain reactions with a reference Ct ; wherein the result of the PCR assay is to be determined positive when said measured Ct is less than said reference Ct or when a unimodal dissociation curve of PCR product is observed and said measured Ct is less than said reference Ct, and the result of the PCR assay is to be determined negative when said measured Ct is greater than or equal to said reference Ct or no logarithmic amplification curve is observed; and whereby, said sample is to be determined positive for presence of HPV when the PCR assay with any one of said primer pairs gives a positive result. Meanwhile, the presence may also be determined using any other methods for such determination as appropriate and as known to those skilled in the art.

It will be understood that the value of the reference Ct is preset depending on the minimum copy number (sensitivity) of viral DNA intended with the detection. For instance, when the virus copy number in a sample is 10⁶, the measured Ct will generally be 17-18; when the virus copy number in a sample is 10⁵, the measured Ct will generally be 21-22; when the virus copy number in a sample is 10⁴, the measured Ct will generally be 24-25; when the virus copy number in a sample is 10³, the measured Ct will generally be 28-29; when the virus copy number in a sample is 100, the measured Ct will generally be 31-32; when the virus copy number in a sample is 50, the measured Ct will generally be 32-33; and when the virus copy number in a sample is 5-10, the measured Ct will generally be around 34. Thereby, a person skilled in the art is capable of setting an appropriate reference Ct according practical needs in detection. For example, in some embodiments of the present invention, the reference Ct may range from 30 to 35, from 32 to 35 or from 33 to 35, or may be 34 or 35. In an embodiment, the reference Ct is 35.

In the present disclosure, primer pairs refer to the paired forward primer (indicated by "F") and the reverse primer (indicated by "R") in PCR. In order to reduce the number of PCR assays, degenerate primer pairs, i.e., primer pairs each allowing for detection of multiple HPV genotypes, may be used.

In an embodiment, the primer pairs include primers that are capable of detecting at least the 65 HPV genotypes, with degenerate primer pairs being preferred. A degenerate primer pair refers to a mixture of primers that represent all possible sequences encoding each single amino acid, which is usually an isometric mixture. In sequences of degenerate primer pairs, R represents A or G; Y represents C or T; M represents A or C; K represents G or T; S represents C or G; W represents A or T; H represents A, C or T; B represents C, G or T; V represents A, C or G; D represents A, G or T; and N represents A, C, G or T. In the present disclosure, primer pairs having the following sequences are preferred:

| Genotype group | HPV genotypes detectable by degenerate primer pair | Sequences of degenerate primer pair |
|---|---|---|
| **1** | HPV32 | F1: CWGGWRCWGAYAATAGRGAAAATG (SEQ ID NO: 1) |
| | HPV42 | |
| | | R1: YRCCATATCMCCATCCTGWATA (SEQ ID NO: 2) |
| | HPV54 | |
| 2 | HPV3 | F2: GCRACAAWCTTGAGGACCARAT |
| | HPV10 | (SEQ ID NO: 3) |
| | HPV28 | R2: YGTGTGCTAGATACRGGTTCAAATG (SEQ ID NO: 4) |
| | HPV29 | |
| | HPV77 | |
| | HPV78 | |
| | HPV94 | |
| | HPV117 | |
| | HPV125 | |
| | HPV160 | |
| 3 | HPV83 | F3: TGCGTGCCAGGAGABGYTAATAGAC (SEQ ID NO: 5) |
| | HPV102 | |
| | | R3: RKGTCCTGGGTRCTAGATAC (SEQ ID NO: 6) |
| | HPV89 | |
| 4 | HPV61 | F4: CCYGAKGTGRTTGACCTACA (SEQ ID NO: 7) |
| | HPV62 | |
| | HPV72 | R4: VVHRTYRGCCATGTCTCTTATG (SEQ ID NO: 8) |
| | HPV81 | |
| 5 | HPV84 | F5: TGCWGAGCCBTATGGCGATTG (SEQ ID NO: 9) |
| | HPV86 | |
| | HPV87 | R5: ACMCCAAAATTCCACTCATC (SEQ ID NO: 10) |
| | HPV114 | |
| 6 | HPV2 | F6: TGTGGCCTAAACGACGTAAAC (SEQ ID NO: 11) |
| | HPV27 | |
| | | R6: CGCGTGACATAGACATCCGTACTG (SEQ ID NO: 12) |
| | HPV57 | |
| 7 | HPV71 | F7: GGAGAMGTTRCTAGAACTGTATGA (SEQ ID NO: 13) |
| | HPV90 | |
| | | R7: GGYDCTGYGTCCCACATTTC (SEQ ID NO: 14) |
| | HPV106 | |
| 8 | HPV26 | F8: GTGCCAGGAGAAAATACTAGACT (SEQ ID NO: 15) |
| | HPV51 | |
| | HPV69 | R8: YSCYGBAGGTACTAGATACA (SEQ ID NO: 16) |
| | HPV82 | |
| 9 | HPV30 | F9: YGCCTATGGBGATTCTATGTG (SEQ ID NO: 17) |
| | HPV53 | |
| | HPV56 | R9: CCACTAGGHGTAGCAACATATAC (SEQ ID NO: 18) |
| | HPV66 | |
| 10 | HPV18 | F10: GGCMCAGGGYCATAACAATGGT (SEQ ID NO: 19) |
| | HPV45 | |
| | | R10: DCGWCCAAGRGGATATTGAT (SEQ ID NO: 20) |
| | HPV97 | |
| 11 | HPV39 | F11: GCWGATGTRTATGGRGACAGTATGT (SEQ ID NO: 21) |
| | HPV68 | |
| | | R11: RGGCTTRTTAAAYAACTGGGARTC (SEQ ID NO: 22) |
| | HPV70 | |
| 12 | HPV59 | F12: TGTCCACCAATGCTTATTACA (SEQ ID NO: 23) |
| | HPV85 | |
| | | R12: CCTCTTCCTSGTKCAAATCTAATC (SEQ ID NO: 24) |
| 13 | HPV7 | F13: GATGGWAACCCAACTAGYATAG (SEQ ID NO: 25) |
| | HPV40 | |
| | | R13: CCGTTGCTGTCAAATGGAAATG (SEQ ID NO: 26) |
| 14 | HPV43 | F14: ASYGAAGTATCCTCTGTTGCGTCTA (SEQ ID NO: 27) |
| | HPV91 | |
| | | R14: TCGAATCGCACTCGCTTTCTA (SEQ ID NO: 28) |
| 15 | HPV16 | F15: GTTGTWAGCACKGATGAATATG (SEQ ID NO: 29) |
| | HPV31 | |
| | | R15: CCACTAATRCCYACACCYAATG (SEQ ID NO: 30) |
| | HPV35 | |
| 16 | HPV33 | F16: GACGTGARCARATGTTTGTTAG (SEQ ID NO: 31) |
| | HPV58 | |
| | HPV52 | R16: GGWGGKGTWARRCCAAATTG (SEQ ID NO: 32) |
| | HPV67 | |
| 17 | HPV6 | F17: TRCACGYARACGCCGTAAAC (SEQ ID NO: 33) |
| | HPV11 | |
| | HPV13 | |
| | | R17: KKCCYACWGCAAGTAGTCTA (SEQ ID NO: 34) |
| | HPV44 | |
| | HPV74 | |
| 18 | HPV34 | F18: GGCMWTRTTAGATGATGCWACA (SEQ ID NO: 35) |
| | HPV73 | |
| | | R18: CACGTTCATACAGTTCTAGGA (SEQ ID NO: 36) |
| | HPV177 | |

In some embodiments, the sample is selected from the group consisting of cells, blood or secretions. In some embodiments, the sample is cells, for example, stem cells, and particularly MSCs.

The present invention further provides a kit for detection of human papillomavirus. The kit may comprise one or more reagents capable of detecting at least the 65 HPV genotypes as defined above. The reagents may contain or consist of primer pairs, preferably degenerate primer pairs. In an embodiment, the kit of the invention may include one or more primer pairs selected from Table 1. The kit may be one for PCR-based assays. For example, the kit may further comprise other components to form a complete polymerase chain reaction system. A polymerase chain reaction system may further comprise other components such as dNTPs, DNA polymerases, buffers, etc., in addition to a template and primers, as well known to and can be determined as appropriate by those skilled in the art. The kit may further include negative and positive controls. The kit may be one for assays on a sample selected from the group consisting of cells, blood or secretions, wherein the cells may be stem cells, for example, MSCs.

The present invention further provides a set of primer pairs for detecting HPV, including one or more primer pairs selected from Table 1. In an embodiment, provided is a set of primer pairs capable of detecting at least the 65 HPV genotypes as defined above, including or consisting of all the primer pairs as listed in Table 1.

The present invention will be further described in details with reference to the examples below. It should be understood that these examples are merely provided for the purpose of illustration, with no intention to limit the scope of the invention in any sense.

### Example 1-Phylogenetic tree analysis of DNA sequences of L1 region of different HPV genotypes

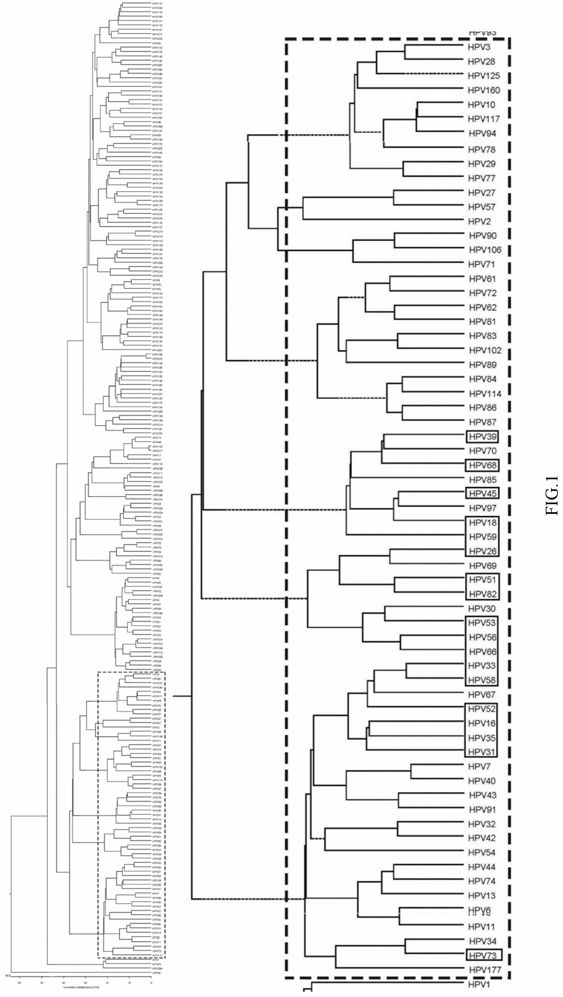

1) Sequences of the L1 region for all the HPV genotypes that have been characterized with defined DNA sequences so far were obtained from the Master Database of Human Papillomaviruses (Human Reference clones - hpvcenter: https://www.hpvcenter.se/), the DNA database (GenBank: https://www.ncbi.nlm.nih.gov/nuccorel) established by National Center for Biotechnology Information (NCBI), the Japan DNA Database (DDBJ: https://www.ddbj.nig.ac.jp),), and the European DNA Database (EMBI: https://www.embl.org)), and the DNA sequences of L1 region of 222 different HPV genotypes were downloaded.
2) The obtained DNA sequences of L1 region of the 222 different HPV genotypes were imported into the software MegAlign (https://www.dnastar.com/) for phylogenetic tree analysis, including alignment of the DNA sequences of L1 region.
3) By analyzing the phylogenetic tree (see FIG. 1 for the result), the inventors found the large branch covering all the high-risk genotypes, i.e., HPV16, 18, 31, 33, 35, 39, 45, 51, 52, 56, 58, 59 and 68, and the medium-risk genotypes, i.e., HPV26, 53, 66, 73 and 82, and further identified all the HPV genotypes covered under this large branch. A total of 65 HPV genotypes were thus identified as follows: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73, and HPV177.

### Example 2-Phylogenetic tree analysis of DNA sequences of L2 region of different HPV genotypes

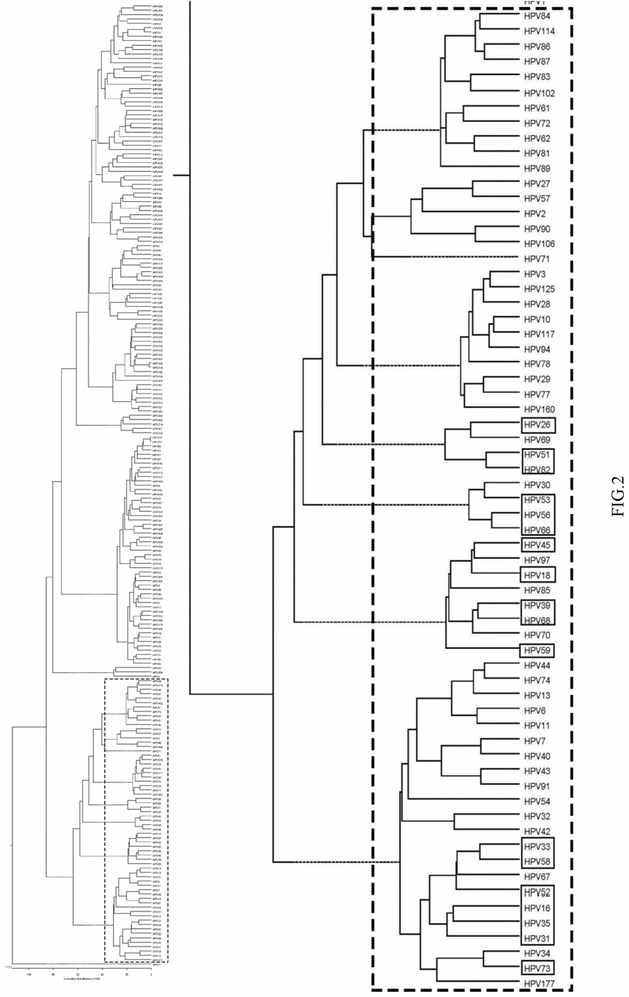

1) Sequences of the L2 region for all the HPV genotypes that have been characterized with defined DNA sequences so far were obtained from the Master Database of Human Papillomaviruses (Human Reference clones - hpvcenter: https://www.hpvcenter.se/), the DNA database (GenBank: https://www.ncbi.nlm.nih.gov/nuccorel) established by National Center for Biotechnology Information (NCBI), the Japan DNA Database (DDBJ: https://www.ddbj.nig.ac.jp),), and the European DNA Database (EMBI: https://www.embl.org)), and the DNA sequences of L2 region of 221 different HPV genotypes were downloaded.
2) The obtained DNA sequences of L2 region of the 221 HPV genotypes were imported into the software MegAlign (https://www.dnastar.com/) for phylogenetic tree analysis, including alignment of the DNA sequences of L2 region.
3) By analyzing the phylogenetic tree (see FIG. 2 for the result), the inventors found the large branch covering all the high-risk genotypes, i.e., HPV16, 18, 31, 33, 35, 39, 45, 51, 52, 56, 58, 59 and 68, and the medium-risk genotypes, i.e., HPV26, 53, 66, 73 and 82, and further identified all the HPV genotypes covered under this large branch. A total of 65 HPV genotypes were thus identified as follows: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73, and HPV177.

### Example 3-Phylogenetic tree analysis of DNA sequences of E1 region of different HPV genotypes

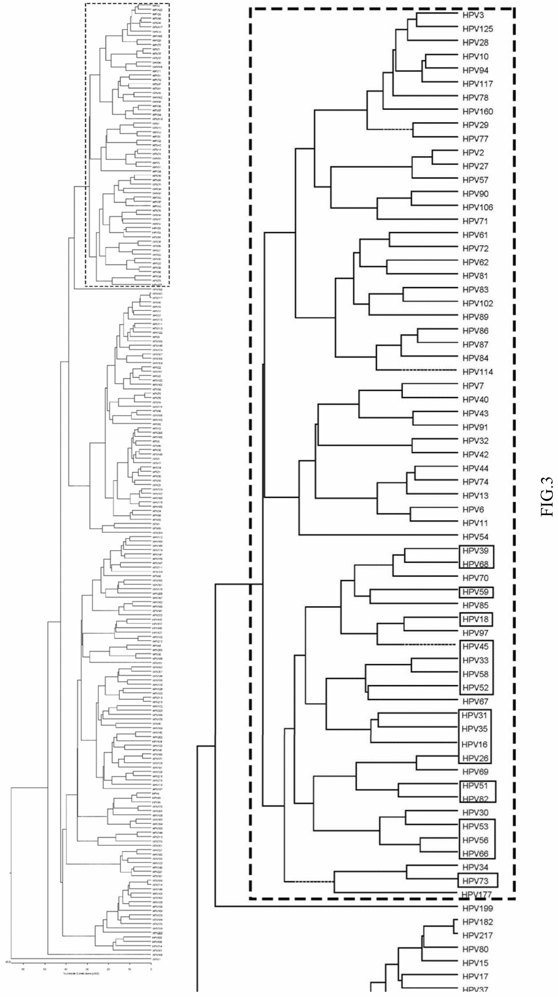

1) Sequences in the E1 region for all the HPV genotypes that have been characterized with defined DNA sequences so far were obtained from the Master Database of Human Papillomaviruses (Human Reference clones - hpvcenter: https://www.hpvcenter.se/), the DNA database (GenBank: https://www.ncbi.nlm.nih.gov/nuccorel) established by National Center for Biotechnology Information (NCBI), the Japan DNA Database (DDBJ: https://www.ddbj.nig.ac.jp),), and the European DNA Database (EMBI: https://www.embl.org)), and the DNA sequences of E1 region of 220 HPV genotypes were downloaded.
2) The obtained DNA sequences of E1 region of the 220 HPV genotypes were imported into the software MegAlign (https://www.dnastar.com/) for phylogenetic tree analysis, including alignment of the DNA sequences of E1 region.
3) By analyzing the phylogenetic tree (see FIG. 3 for the result), the inventors found the large branch covering all the high-risk genotypes, i.e., HPV16, 18, 31, 33, 35, 39, 45, 51, 52, 56, 58, 59 and 68, and the medium-risk genotypes, i.e., HPV26, 53, 66, 73 and 82, and further identified all the HPV genotypes covered under this large branch. A total of 65 HPV genotypes were thus identified as follows: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73, and HPV177.

### Example 4-Phylogenetic tree analysis of DNA sequences of E6 region of different HPV genotypes

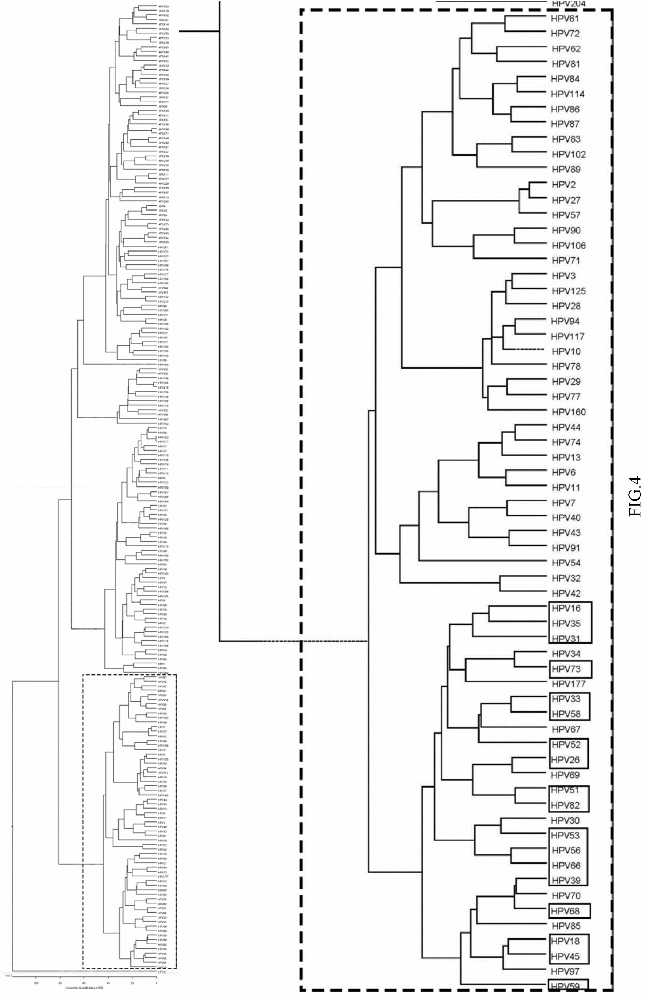

1) Sequences of the E6 region for all the HPV genotypes that have been characterized with defined DNA sequences so far were obtained from the Master Database of Human Papillomaviruses (Human Reference clones - hpvcenter: https://www.hpvcenter.se/), the DNA database (GenBank: https://www.ncbi.nlm.nih.gov/nuccorel) established by National Center for Biotechnology Information (NCBI), the Japan DNA Database (DDBJ: https://www.ddbj.nig.ac.jp,), and the European DNA Database (EMBI: https://www.embl.org)), and the DNA sequences of E6 region of 214 HPV genotypes were downloaded.
2) The obtained DNA sequences of E6 region of the 214 HPV genotypes were imported into the software MegAlign (https://www.dnastar.com/) for phylogenetic tree analysis, including alignment of the DNA sequences of E6 region.
3) By analyzing the phylogenetic tree (see FIG. 4 for the result), the inventors found the large branch covering all the high-risk genotypes, i.e., HPV16, 18, 31, 33, 35, 39, 45, 51, 52, 56, 58, 59 and 68, and the medium-risk HPVs genotypes, i.e., HPV26, 53, 66, 73 and 82, and further identified all the HPV genotypes covered under this large branch. A total of 65 HPV genotypes were thus identified as follows: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73, and HPV177.

### Example 5 - Verification of degenerate primer pairs for 65 HPV genotypes

### (I) Design and synthesis of primers

Whole genomes of the 65 HPV genotypes were aligned, whereby the 65 genotypes were divided into 18 groups; and then, a degenerate primer pair was designed for HPV genotypes of the same group (see Table 3). The degenerate primer pairs were synthesized, for which the threshold of the sensitivity was set to be 5-100 copies.

### (II) Settings of control and test groups

Negative control 1: water control;
Negative control 2: gDNA negative control;
Positive control: gDNA positive control; and
Test group: gDNA templates plus synthesized fragment templates of HPV genotypes. Sequences of said fragment templates of HPV genotypes were as summarize in Table 2 below, wherein the Gene IDs came from the data published by the International Human Papillomavirus (HPV) Reference Center (https://www.hpvcenter.se/human_reference_clones/).

The templates in the gDNA positive control and the gDNA negative control were genomic DNAs (also referred to "gDNA templates" herein) extracted from human cells without HPV infection., which were purchased from Shanghai SXBIO Biotech Co., Ltd. under the name of "Human Marrow Mesenchymal Stem Cell Genomic DNA (HMSC-bm gDNA)".

The primers added to the gDNA positive control were human β-globin primers (F: GAAGAGCCAAGGACAGGTAC, R: CAACTTCATCCACGTTCACC). The primers added to the water control and the gDNA negative control were those listed in Table 3, wherein each PCR system contains one primer pair to thereby create 18 water control PCR systems in parallel each comprising a distinct primer pair and 18 gDNA negative control PCR systems in parallel each comprising a distinct primer pair. In the test group, each of the 65 HPV genotype templates was reacted with the primer pair assigned to the group it belongs to at the specified copy number as set forth in Table 3 to thereby create multiplex PCR assays comprising reactions of the 65 different HPV genotype templates in parallel.

**Table 1 Fragment templates of HPV genotypes**

| HPV genotype | Gene ID | Position of the Start of fragment template | Position of the End of fragment template |
|---|---|---|---|
| HPV32 | X74475 | 6233 | 6513 |
| HPV42 | M73236 | 6199 | 6480 |
| HPV54 | U37488 | 5967 | 6254 |
| HPV3 | X74462 | 2744 | 3396 |
| HPV10 | X74465 | 2796 | 3451 |
| HPV28 | U31783 | 2735 | 3390 |
| HPV29 | U31784 | 2744 | 3402 |
| HPV77 | Y15175 | 2747 | 3405 |
| HPV78 | AB793779 | 2634 | 3149 |
| HPV94, | AJ620211 | 2795 | 3450 |
| HPV117 | GQ246950 | 2793 | 3451 |
| HPV125 | FN547152 | 2640 | 3292 |
| HPV160 | AB745694 | 2746 | 3395 |
| HPV83 | AF151983 | 2591 | 3287 |
| HPV102 | DQ080083 | 2588 | 3284 |
| HPV89 | AF436128 | 2603 | 3307 |
| HPV61 | U31793 | 516 | 872 |
| HPV62 | AY395706 | 421 | 780 |
| HPV72 | X94164 | 522 | 893 |
| HPV81 | AJ620209 | 540 | 905 |
| HPV84 | AF293960 | 6337 | 6937 |
| HPV86 | AF349909 | 6330 | 6933 |
| HPV87 | AJ400628 | 6427 | 7033 |
| HPV114 | GQ244463 | 6573 | 7188 |
| HPV2 | X55964 | 5698 | 5928 |
| HPV27 | X74473 | 5672 | 5902 |
| HPV57 | X55965 | 5659 | 5889 |
| HPV71 | AB040456 | 2689 | 3070 |
| HPV90 | AY057438 | 2592 | 2973 |
| HPV106 | DQ080082 | 2628 | 3009 |
| HPV26 | X74472 | 2715 | 3398 |
| HPV51 | M62877 | 2699 | 3379 |
| HPV69 | AB027020 | 2711 | 3394 |
| HPV82 | AB027021 | 2725 | 3405 |
| HPV30 | X74474 | 6321 | 6591 |
| HPV53 | X74482 | 6318 | 6588 |
| HPV56 | X74483 | 6263 | 6533 |
| HPV66 | U31794 | 6313 | 6583 |
| HPV18 | X05015 | 6507 | 7063 |
| HPV45 | X74479 | 6511 | 7064 |
| HPV97 | DQ080080 | 6387 | 6943 |
| HPV39 | M62849 | 6304 | 6619 |
| HPV68 | X67161 | 858 | 1173 |
| HPV70 | U21941 | 6248 | 6563 |
| HPV59 | X77858 | 2478 | 2782 |
| HPV85 | AF131950 | 2532 | 2836 |
| HPV7 | X74463 | 2435 | 2707 |
| HPV40 | X74478 | 2438 | 2709 |
| HPV43 | AJ620205 | 3303 | 3530 |
| HPV91 | AF419318 | 3370 | 3594 |
| HPV16 | K02718 | 5647 | 6042 |
| HPV31 | J04353 | 5562 | 5960 |
| HPV35 | X74477 | 5611 | 6006 |
| HPV33 | M12732 | 6295 | 6854 |
| HPV58 | D90400 | 6344 | 6903 |
| HPV52 | X74481 | 6353 | 6918 |
| HPV67 | D21208 | 6320 | 6879 |
| HPV6 | X00203 | 5698 | 5974 |
| HPV11 | M14119 | 5680 | 5956 |
| HPV13 | X62843 | 5651 | 5927 |
| HPV44 | U31788 | 5603 | 5879 |
| HPV74 | AF436130 | 5500 | 5776 |
| HPV34 | X74476 | 2348 | 2849 |
| HPV73 | X94165 | 2356 | 2857 |
| HPV177 | KR816168 | 2315 | 2816 |

### (III) Preparation for nucleic acid amplification

1) Place a 96-well PCR plate on ice for later use.
2) Onto the plate, PCR systems of the following compositions for the control and the test groups were added:

| | Volume | | | |
|---|---|---|---|---|
| Component | Water control | gDNA negative control | gDNA positive control | Test group |
| Fragment templates of HPV genotypes | - | - | - | 1µL |
| gDNA | - | 50ng | 50ng | 50ng |
| 2xTransStart ^{R} Top Green qPCR | 10µL | | | |

| SuperMix | | | | |
|---|---|---|---|---|
| Forward primer (10 µM) | 0.5µL | | | |
| Reverse primer (10 µM) | 0.5µL | | | |
| Distilled water, making up to | 20µL | | | |

3) The plate was film sealed, centrifuged at 2000 rpm for 1 minute, and then transferred to the amplification and detection zoon.

### (IV) PCR amplification

1) The PCR amplification parameters were as follows: denaturation at 94°C for 30 seconds, followed by 40 cycles of 94°C for 5 seconds, 55°C for 15 seconds, and 72°C for 35 seconds.
2) Fluorescence value was measured at 72°C, and the Ct value was determined from the fluorescence curve, i.e., the sigmoid logarithmic amplification curve.
3) Quality control is shown in the table below:

| Sample | Ct value | Result |
|---|---|---|
| Water control | No logarithmic amplification curve | HPV negative |
| gDNA negative control | No logarithmic amplification curve | HPV negative |
| gDNA positive control | Ct<35 | gDNA positive |

### (V) Result analysis

In this study, the reference threshold Ct was set at 35. The result was determined positive, when a unimodal dissociation curve of PCR product was observed and the Ct value was less than 35; and the result was determined negative, when the Ct value was greater than or equal to 35 or no logarithmic amplification curve was observed.

When the results of the water control and the gDNA negative control were negative while the result of the gDNA positive control was positive, and the result of the test was positive, the degenerate primer pair were determined effective to amplify the HPV genotypes of the group to which the pair were assigned, and the result of verification was defined as positive. The verification results of degenerate primer pairs for 65 HPV genotypes were as summarized in Table 3.

**Table 2 Verification of degenerate primer pairs corresponding to HPVs in different group**

| Group | HPV genotype | Template copy number | Sequences of degenerate primer pairs | Verification result |
|---|---|---|---|---|
| 1 | HPV32 | 50 | F1: CWGGWRCWGAYAATAGRGAAAATG (SEQ ID NO: 1) | Positive |
| | HPV42 | 10 | | Positive |
| | HPV54 | 10 | R1: YRCCATATCMCCATCCTGWATA (SEQ ID NO: 2) | Positive |
| 2 | HPV3 | 10 | F2: GCRACAAWCTTGAGGACCARAT (SEQ ID NO: 3) | Positive |
| | HPV10 | 50 | | Positive |
| | HPV28 | 10 | R2: YGTGTGCTAGATACRGGTTCAAATG | Positive |
| | HPV29 | 5 | (SEQ ID NO: 4) | Positive |
| | HPV77 | 50 | | Positive |
| | HPV78 | 10 | | Positive |
| | HPV94 | 10 | | Positive |
| | HPV117 | 50 | | Positive |
| | HPV125 | 10 | | Positive |
| | HPV160 | 10 | | Positive |
| 3 | HPV83 | 50 | F3: TGCGTGCCAGGAGABGYTAATAGAC (SEQ ID NO: 5) | Positive |
| | HPV102 | 5 | | Positive |
| | HPV89 | 10 | R3: RKGTCCTGGGTRCTAGATAC (SEQ ID NO: 6) | Positive |
| 4 | HPV61 | 50 | F4: CCYGAKGTGRTTGACCTACA (SEQ ID NO: 7) | Positive |
| | HPV62 | 100 | | Positive |
| | HPV72 | 10 | R4: VVHRTYRGCCATGTCTCTTATG (SEQ ID NO: 8) | Positive |
| | HPV81 | 100 | | Positive |
| 5 | HPV84 | 10 | F5: TGCWGAGCCBTATGGCGATTG (SEQ ID NO: 9) | Positive |
| | HPV86 | 10 | | Positive |
| | HPV87 | 50 | R5: ACMCCAAAATTCCACTCATC (SEQ ID NO: 10) | Positive |
| | HPV114 | 50 | | Positive |
| 6 | HPV2 | 10 | F6: TGTGGCCTAAACGACGTAAAC (SEQ ID NO: 11) | Positive |
| | HPV27 | 10 | | Positive |
| | HPV57 | 10 | R6: CGCGTGACATAGACATCCGTACTG (SEQ ID NO: 12) | Positive |
| 7 | HPV71 | 100 | F7: GGAGAMGTTRCTAGAACTGTATGA (SEQ ID NO: 13) | Positive |
| | HPV90 | 100 | | Positive |
| | HPV106 | 100 | R7: GGYDCTGYGTCCCACATTTC (SEQ ID NO: 14) | Positive |
| 8 | HPV26 | 100 | F8: GTGCCAGGAGAAAATACTAGACT (SEQ ID NO: 15) | Positive |
| | HPV51 | 50 | | Positive |
| | HPV69 | 50 | R8: YSCYGBAGGTACTAGATACA (SEQ ID NO: 16) | Positive |
| | HPV82 | 50 | | Positive |
| 9 | HPV30 | 50 | F9: YGCCTATGGBGATTCTATGTG (SEQ ID NO: 17) | Positive |
| | HPV53 | 50 | | Positive |
| | HPV56 | 10 | R9: CCACTAGGHGTAGCAACATATAC (SEQ ID NO: 18) | Positive |
| | HPV66 | 50 | | Positive |
| 10 | HPV18 | 50 | F10: GGCMCAGGGYCATAACAATGGT (SEQ ID NO: 19) | Positive |
| | HPV45 | 100 | | Positive |
| | HPV97 | 50 | R10: DCGWCCAAGRGGATATTGAT (SEQ ID NO: 20) | Positive |
| 11 | HPV39 | 50 | F11: GCWGATGTRTATGGRGACAGTATGT (SEQ ID NO: 21) | Positive |
| | HPV68 | 50 | | Positive |
| | HPV70 | 50 | R11: RGGCTTRTTAAAYAACTGGGARTC (SEQ ID NO: 22) | Positive |
| 12 | HPV59 | 5 | F12: TGTCCACCAATGCTTATTACA (SEQ ID NO: 23) | Positive |
| | | | | |
| | HPV85 | 10 | R12: CCTCTTCCTSGTKCAAATCTAATC (SEQ ID NO: 24) | Positive |
| 13 | HPV7 | 100 | F13: GATGGWAACCCAACTAGYATAG (SEQ ID NO: 25) | Positive |
| | | | | |
| | HPV40 | 10 | R13: CCGTTGCTGTCAAATGGAAATG (SEQ ID NO: 26) | Positive |
| 14 | HPV43 | 10 | F14: ASYGAAGTATCCTCTGTTGCGTCTA (SEQ ID NO: 27) | Positive |
| | | | | |
| | HPV91 | 10 | R14: TCGAATCGCACTCGCTTTCTA (SEQ ID NO: 28) | Positive |
| 15 | HPV16 | 50 | F15: GTTGTWAGCACKGATGAATATG (SEQ ID NO: 29) | Positive |
| | HPV31 | 50 | | Positive |
| | HPV35 | 10 | R15: CCACTAATRCCYACACCYAATG (SEQ ID NO: 30) | Positive |
| 16 | HPV33 | 10 | F16: GACGTGARCARATGTTTGTTAG (SEQ ID NO: 31) | Positive |
| | HPV58 | 10 | | Positive |
| | HPV52 | 50 | R16: GGWGGKGTWARRCCAAATTG (SEQ ID NO: 32) | Positive |
| | HPV67 | 50 | | Positive |
| 17 | HPV6 | 50 | F17: TRCACGYARACGCCGTAAAC (SEQ ID NO: 33) | Positive |
| | HPV11 | 10 | | Positive |
| | HPV13 | 50 | | Positive |
| | | | R17: KKCCYACWGCAAGTAGTCTA (SEQ ID NO: 34) | |
| | HPV44 | 50 | | Positive |
| | HPV74 | 10 | | Positive |
| 18 | HPV34 | 10 | F18: GGCMWTRTTAGATGATGCWACA (SEQ ID NO: 35) | Positive |
| | HPV73 | 10 | | Positive |
| | HPV177 | 10 | R18: CACGTTCATACAGTTCTAGGA (SEQ ID NO: 36) | Positive |

Specifically, no sigmoid logarithmic amplification curve was observed with the 18 PCR reactions of the water control and the 18 PCR reactions of the gDNA negative control, and therefore, the results for these controls were negative. The gDNA positive control showed a unimodal dissolution curve of PCR product and a Ct value of 22 that was less than 35, and therefore, the result was positive. All the 65 PCR products in the test group each showed a unimodal dissolution curve and a Ct ranging from 31 to 34 that were all less than 35. Therefore, the results of verification for the primer pairs were all positive.

### Example 6 - Detection of HPV in a cell sample

### (I) Sample processing and DNA extraction

1) MSCs derived from hair follicles were resuspended in PBS to a concentration of 1×10⁶/ml, and 5-10 µL of the suspension was added into 250 µL of lysate, i.e., QuickExtract^{™} DNA extraction solution.
2) The resultant mixture was oscillated for 15 seconds, followed by a water bath at 65°C for 6 minutes, then oscillated for another 15 seconds, followed by a water bath at 98°C for 2 minutes after a short-spin, and then oscillated for 15 seconds, followed by a short-spin to obtain a supernatant of cell lysate. The supernatant of lysate was used as fresh or stored at -20°C.

In this example, the samples were added with HPV viruses to mimic viral infection or contamination so as to test on feasibility of the method for detecting HPV infection in samples.

### (II) Settings of control and test groups

Negative Control: 10 µL PBS+250 µL QuickExtract^{™} DNA extraction solution
Positive control: supernatant of MSC lysate
Test group: supernatant of MSC lysate with addition of synthesized fragment templates of genotypes HPV61, HPV84, and HPV26 (100 copies for each).

The primers added into the positive control were human β-globin primers (F: GAAGAGCCAAGGACAGGTAC, R: CAACTTCATCCACGTTCACC). The primers added into the negative control were the 18 degenerate primer pairs shown in Table 4, whereby 18 PCR systems were formed for respective amplifications with these primer pairs. The primers added into the test group were the 18 degenerate primer pairs shown in Table 4, whereby 18 PCR systems were formed for respective amplifications with these primer pairs.

### (III) Preparation for nucleic acid amplification

1) A 96-well PCR plate was placed on ice for later use.
2) Onto the plate, PCR systems of the following compositions for the control group and the test group were added:

| | Volume | | |
|---|---|---|---|
| Component | Negative control | Positive control | Test group |
| Fragment templates of genotypes HPV61, HPV84 and HPV26 | - | - | 1µL |
| PBS + QuickExtractTM DNA extraction solution | 2µL | - | - |
| Supernatant of MSC lysate | - | 2µL | 2µL |
| 2xTransStartR Top Green qPCR SuperMix | 10µL | | |
| Forward primer (10 µM) | 0.5µL | | |
| Reverse primer (10 µM) | 0.5µL | | |
| Distilled water, making up to | 20µL | | |

3) The plate was film sealed, centrifuged at 2000 rpm for 1 minute, and then transferred to the amplification and detection zoon.

### (IV) PCR amplification

1) The PCR amplification parameters were as follows: denaturation at 94°C for 30 seconds, followed by 40 cycles of 94°C for 5 seconds, 55°C for 15 seconds, and 72°C for 35 seconds.
2) Fluorescence value was measured at 72°C, and the result of the Ct value was determined from the fluorescence curve, i.e., the sigmoid logarithmic amplification curve.
3) Quality control is shown in the table below:

| Sample | Ct value | Result |
|---|---|---|
| Negative control | No logarithmic amplification curve | HPV negative |
| Positive control | Ct<35 | gDNA positive |

### (V) Result analysis

In this study, for the method of the present invention, the reference threshold Ct was set at 35. The result was determined positive, when a unimodal dissociation curve of PCR product was observed and the Ct value was less than 35; and the result was determined negative, when the Ct value was greater than or equal to 35 or no logarithmic amplification curve was observed.

When result of the negative control was negative while the result of positive control positive, and PCR with any one of the primer pairs in the test group gave a positive result, it was determined that a HPV genotype corresponding to the primer pair was detected and marked positive for its presence; and meanwhile, the virus detection result for the sample was also determined positive, which stood as an indication of HPV infection. The test results were as summarized in Table 4.

**Table 4 Detection results of HPV genotypes**

| Group | HPV genotype | Sequences of degenerate primer pair | Test result |
|---|---|---|---|
| 1 | HPV32 | F1: CWGGWRCWGAYAATAGRGAAAATG (SEQ ID NO: 1) | Negative |
| | HPV42 | | |
| | HPV54 | R1: YRCCATATCMCCATCCTGWATA (SEQ ID NO: 2) | |
| 2 | HPV3 | F2: GCRACAAWCTTGAGGACCARAT (SEQ ID NO: 3) | Negative |
| | HPV10 | | |
| | HPV28 | | |
| | HPV29 | | |
| | HPV77 | | |
| | HPV78 | R2: YGTGTGCTAGATACRGGTTCAAATG (SEQ ID NO: 4) | |
| | HPV94, | | |
| | HPV117 | | |
| | HPV125 | | |
| | HPV160 | | |
| 3 | HPV83 | F3: TGCGTGCCAGGAGABGYTAATAGAC (SEQ ID NO: 5) | Negative |
| | HPV102 | | |
| | HPV89 | R3: RKGTCCTGGGTRCTAGATAC (SEQ ID NO: 6) | |
| 4 | HPV61 | F4: CCYGAKGTGRTTGACCTACA (SEQ ID NO: 7) | Positive |
| | HPV62 | | |
| | HPV72 | R4: VVHRTYRGCCATGTCTCTTATG | |
| | HPV81 | (SEQ ID NO: 8) | |
| 5 | HPV84 | F5: TGCWGAGCCBTATGGCGATTG (SEQ ID NO: 9) | Positive |
| | HPV86 | | |
| | HPV87 | R5: ACMCCAAAATTCCACTCATC (SEQ ID NO: 10) | |
| | HPV114 | | |
| 6 | HPV2 | F6: TGTGGCCTAAACGACGTAAAC (SEQ ID NO: 11) | Negative |
| | HPV27 | | |
| | HPV57 | R6: CGCGTGACATAGACATCCGTACTG (SEQ ID NO: 12) | |
| 7 | HPV71 | F7: GGAGAMGTTRCTAGAACTGTATGA (SEQ ID NO: 13) | Negative |
| | HPV90 | | |
| | HPV106 | R7: GGYDCTGYGTCCCACATTTC (SEQ ID NO: 14) | |
| 8 | HPV26 | F8: GTGCCAGGAGAAAATACTAGACT (SEQ ID NO: 15) | Positive |
| | HPV51 | | |
| | HPV69 | R8: YSCYGBAGGTACTAGATACA (SEQ ID NO: 16) | |
| | HPV82 | | |
| 9 | HPV30 | F9: YGCCTATGGBGATTCTATGTG (SEQ ID NO: 17) | Negative |
| | HPV53 | | |
| | HPV56 | R9: CCACTAGGHGTAGCAACATATAC (SEQ ID NO: 18) | |
| | HPV66 | | |
| 10 | HPV18 | F10: GGCMCAGGGYCATAACAATGGT (SEQ ID NO: 19) | Negative |
| | HPV45 | | |
| | HPV97 | R10: DCGWCCAAGRGGATATTGAT (SEQ ID NO: 20) | |
| 11 | HPV39 | F11: GCWGATGTRTATGGRGACAGTATGT (SEQ ID NO: 21) | Negative |
| | HPV68 | | |
| | HPV70 | R11: RGGCTTRTTAAAYAACTGGGARTC (SEQ ID NO: 22) | |
| 12 | HPV59 | F12:TGTCCACCAATGCTTATTACA (SEQ ID NO: 23) | Negative |
| | HPV85 | | |
| | | R12:CCTCTTCCTSGTKCAAATCTAATC (SEQ ID NO: 24) | |
| 13 | HPV7 | F13: GATGGWAACCCAACTAGYATAG (SEQ ID NO: 25) | Negative |
| | HPV40 | | |
| | | R13: CCGTTGCTGTCAAATGGAAATG (SEQ ID NO: 26) | |
| 14 | HPV43 | F14: ASYGAAGTATCCTCTGTTGCGTCTA (SEQ ID NO: 27) | Negative |
| | HPV91 | | |
| | | R14: TCGAATCGCACTCGCTTTCTA (SEQ ID NO: 28) | |
| 15 | HPV16 | F15: GTTGTWAGCACKGATGAATATG (SEQ ID NO: 29) | Negative |
| | HPV31 | | |
| | HPV35 | R15: CCACTAATRCCYACACCYAATG (SEQ ID NO: 30) | |
| 16 | HPV33 | F16: GACGTGARCARATGTTTGTTAG (SEQ ID NO: 31) | Negative |
| | HPV58 | | |
| | HPV52 | R16: GGWGGKGTWARRCCAAATTG (SEQ ID NO: 32) | |
| | HPV67 | | |
| 17 | HPV6 | F17: TRCACGYARACGCCGTAAAC (SEQ ID NO: 33) | Negative |
| | HPV11 | | |
| | HPV13 | | |
| | | R17: KKCCYACWGCAAGTAGTCTA (SEQ ID NO: 34) | |
| | HPV44 | | |
| | HPV74 | | |
| 18 | HPV34 | F18: GGCMWTRTTAGATGATGCWACA (SEQ ID NO: 35) | Negative |
| | HPV73 | | |
| | HPV177 | R18: CACGTTCATACAGTTCTAGGA (SEQ ID NO: 36) | |

Specifically, none of the 18 PCR systems of negative control showed a sigmoid logarithmic amplification curve, and therefore, the results were negative. The positive control showed a unimodal dissolution curve of PCR product and a Ct value of 29 that was less than 35, and therefore, the result was positive. In the test group, PCR products of the primer pairs in groups 4, 5 and 8 showed unimodal dissolution curves, with the Ct values ranging from 31 to 32 that were all less than 35. Therefore, the detection results for these groups were positive. As for the other primer pairs in the test group, no logarithmic amplification curve was observed, and thus, the results were negative.

Based on the above PCR results, the cell sample was determined positive for HPV detection.

### Example 7 - Detection of HPV in a sample of cervical secretion

### (I) Sample processing and DNA extraction

1) Sampled secretion was thoroughly eluted from a cervical sampling brush, and the brush was squeezed to dry on tube wall and then discarded. All fractions of eluate were collected into a 1.5 ml microcentrifuge tube and centrifuged at 13,000 rpm for 10 minutes. The supernatant was discarded to obtain the precipitate at the bottom of the tube. 50 µL of lysate, i.e., the QuickExtract^{™} DNA extraction solution, was added to the sample precipitate.
2) The resultant mixture was oscillated for 15 seconds, followed by a water bath at 65°C for 6 minutes, then oscillated for another 15 seconds, followed by a water bath at 98°C for 2 minutes after a short-spin, and then oscillated for 15 seconds, followed by a short-spin to obtain a supernatant of secretion lysate. The supernatant of lysate was used as fresh or stored at -20°C.

In this example, the sample was added with HPV viruses to mimic viral infection or contamination, so as to test on feasibility of the method for detecting HPV infection in samples.

### (II) Settings of control and test groups

Negative control: QuickExtract^{™} DNA extraction solution
Positive control: supernatant of secretion lysate
Test group: supernatant of secretion lysate with addition of synthesized fragment templates of genotypes HPV42, HPV28, and HPV102 (100 copies for each).

The primers added into the positive control were human β-globin primers (F: GAAGAGCCAAGGACAGGTAC, R: CAACTTCATCCACGTTCACC); and the primers added into the negative control were the 18 degenerate primer pairs shown in Table 5, whereby 18 PCR systems were respectively formed for respective amplifications with these primer pairs. The primers added to the test group were 18 degenerate primer pairs shown in Table 5, whereby 18 PCR systems were formed for respective amplifications with these primer pairs.

### (III) Preparation for nucleic acid amplification

1) A 96-well PCR plate was placed on ice for later use.
2) On to the plate, PCR systems of the following compositions for the control group and the test group were added :

| | Volume | | |
|---|---|---|---|
| Component | Negative control | Positive control | Test group |
| Fragments of genotypes HPV42, HPV28, HPV102 | - | - | 1µL |
| QuickExtractTM DNA extraction solution | 2µL | - | - |
| Supernatant of secretion lysate | - | 2µL | 2µL |
| 2xTransStartR Top Green qPCR SuperMix | 10µL | | |
| Forward primer (10 µM) | 0.5µL | | |
| Reverse primer (10 µM) | 0.5µL | | |
| Distilled water, making up to | 20µL | | |

3) The plate was film sealed, centrifuged at 2000 rpm for 1 minute, and then transferred to an amplification and detection zoon.

### (IV) PCR amplification

1) The PCR amplification parameters were as follows: denaturation at 94°C for 30 seconds, followed by 40 cycles of 94°C for 5 seconds, 55°C for 15 seconds, and 72°C for 35 seconds.
2) Fluorescence value was measured at 72°C, and the result of the Ct value was determined from the fluorescence curve, i.e., the sigmoid logarithmic amplification curve.
3) Quality control is shown in the table below:

| Sample | Ct value | Result |
|---|---|---|
| Negative control | No logarithmic amplification curve | HPV negative |
| Positive control | Ct<35 | gDNA positive |

### (V) Result analysis

In this study, for the method of the present invention, the reference threshold Ct was set at 35. The result was determined positive, when a unimodal dissociation curve of PCR product was observed and the Ct value was less than 35; and the result was determined negative, when the Ct value was greater than or equal to 35 or no logarithmic amplification curve was observed.

When result of the negative control was negative while the result of positive control result positive, and PCR with any one of the primer pairs in the test group gave a positive result, it was determined that a HPV genotype corresponding to the primer pair was detected and marked as positive for its presence; and meanwhile, the virus detection result for the sample was also determined positive, which stood as an indication of HPV infection. The test results were as summarized in Table 5.

**Table 5 Detection results of HPV genotypes**

| Group | HPV genotype | Sequences of degenerate primer pair | Test result |
|---|---|---|---|
| 1 | HPV32 | F1: CWGGWRCWGAYAATAGRGAAAATG (SEQ ID NO: 1) | Positive |
| | HPV42 | | |
| | | R1: YRCCATATCMCCATCCTGWATA (SEQ ID NO: 2) | |
| | HPV54 | | |
| 2 | HPV3 | F2: GCRACAAWCTTGAGGACCARAT (SEQ ID NO: 3) | Positive |
| | HPV10 | | |
| | HPV28 | | |
| | HPV29 | | |
| | HPV77 | | |
| | HPV78 | R2: YGTGTGCTAGATACRGGTTCAAATG (SEQ ID NO: 4) | |
| | HPV94, | | |
| | HPV117 | | |
| | HPV125 | | |
| | HPV160 | | |
| 3 | HPV83 | F3: TGCGTGCCAGGAGABGYTAATAGAC (SEQ ID NO: 5) | Positive |
| | HPV102 | | |
| | | R3: RKGTCCTGGGTRCTAGATAC (SEQ ID NO: 6) | |
| | HPV89 | | |
| 4 | HPV61 | F4: CCYGAKGTGRTTGACCTACA (SEQ ID NO: 7) | Negative |
| | HPV62 | | |
| | HPV72 | R4: VVHRTYRGCCATGTCTCTTATG (SEQ ID NO: 8) | |
| | HPV81 | | |
| 5 | HPV84 | F5: TGCWGAGCCBTATGGCGATTG (SEQ ID NO: 9) | Negative |
| | HPV86 | | |
| | HPV87 | R5: ACMCCAAAATTCCACTCATC (SEQ ID NO: 10) | |
| | HPV114 | | |
| 6 | HPV2 | F6: TGTGGCCTAAACGACGTAAAC (SEQ ID NO: 11) | Negative |
| | HPV27 | | |
| | | R6: CGCGTGACATAGACATCCGTACTG (SEQ ID NO: 12) | |
| | HPV57 | | |
| 7 | HPV71 | F7: GGAGAMGTTRCTAGAACTGTATGA (SEQ ID NO: 13) | Negative |
| | HPV90 | | |
| | HPV106 | R7: GGYDCTGYGTCCCACATTTC(SEQ ID NO: 14) | |
| 8 | HPV26 | F8: GTGCCAGGAGAAAATACTAGACT (SEQ ID NO: 15) | Negative |
| | HPV51 | | |
| | HPV69 | R8: YSCYGBAGGTACTAGATACA (SEQ ID NO: 16) | |
| | HPV82 | | |
| 9 | HPV30 | F9: YGCCTATGGBGATTCTATGTG (SEQ ID NO: 17) | Negative |
| | HPV53 | | |
| | HPV56 | R9: CCACTAGGHGTAGCAACATATAC (SEQ ID NO: 18) | |
| | HPV66 | | |
| 10 | HPV18 | F10: GGCMCAGGGYCATAACAATGGT (SEQ ID NO: 19) | Negative |
| | HPV45 | | |
| | | R10: DCGWCCAAGRGGATATTGAT (SEQ ID NO: 20) | |
| | HPV97 | | |
| 11 | HPV39 | F11: GCWGATGTRTATGGRGACAGTATGT (SEQ ID NO: 21) | Negative |
| | HPV68 | | |
| | | R11: RGGCTTRTTAAAYAACTGGGARTC (SEQ ID NO: 22) | |
| | HPV70 | | |
| 12 | HPV59 | F12: TGTCCACCAATGCTTATTACA (SEQ ID NO: 23) | Negative |
| | HPV85 | R12: CCTCTTCCTSGTKCAAATCTAATC (SEQ ID NO: 24) | |
| 13 | HPV7 | F13: GATGGWAACCCAACTAGYATAG (SEQ ID NO: 25) | Negative |
| | HPV40 | | |
| | | R13: CCGTTGCTGTCAAATGGAAATG (SEQ ID NO: 26) | |
| 14 | HPV43 | F14: ASYGAAGTATCCTCTGTTGCGTCTA(SEQ ID NO: 27) | Negative |
| | HPV91 | R14: TCGAATCGCACTCGCTTTCTA (SEQ ID NO: 28) | |
| 15 | HPV16 | F15: GTTGTWAGCACKGATGAATATG (SEQ ID NO: 29) | Negative |
| | HPV31 | | |
| | | R15: CCACTAATRCCYACACCYAATG (SEQ ID NO: 30) | |
| | HPV35 | | |
| 16 | HPV33 | F16: GACGTGARCARATGTTTGTTAG (SEQ ID NO: 31) | Negative |
| | HPV58 | | |
| | HPV52 | R16: GGWGGKGTWARRCCAAATTG (SEQ ID NO: 32) | |
| | HPV67 | | |
| 17 | HPV6 | F17: TRCACGYARACGCCGTAAAC (SEQ ID NO: 33) | Negative |
| | HPV11 | | |
| | HPV13 | | |
| | | R17: KKCCYACWGCAAGTAGTCTA (SEQ ID NO: 34) | |
| | HPV44 | | |
| | HPV74 | | |
| 18 | HPV34 | F18: GGCMWTRTTAGATGATGCWACA (SEQ ID NO: 35) | Negative |
| | HPV73 | | |
| | | R18: CACGTTCATACAGTTCTAGGA (SEQ ID NO: 36) | |
| | HPV177 | | |

Specifically, none of the 18 PCR systems of negative control showed a sigmoid logarithmic amplification curve, and therefore, the results were negative. The positive control showed a unimodal dissolution curve of PCR product and a Ct value of 30 which was less than 35, and therefore, the result was positive. In the test group, PCR products of the primer pairs in groups 1, 2 and 3 showed unimodal dissolution curves, with the Ct values ranging from 31 to 32, which were all less than 35, and therefore, the detection results were positive. As for the other primer pairs in the test group, no logarithmic amplification curve was observed, and thus, the results were negative.

Based on the above PCR results, it was determined that the cervical secretion samples were positive for HPV presence in the detection.

## Claims

1. A method for detecting presence of human papillomavirus (HPV) in a sample, comprising the step of detecting presence of HPVs of at least the following 65 genotypes: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73, and HPV177.

2. The method according to claim 1, wherein, the presence of said genotypes is detected via polymerase chain reaction (PCR) assays and the presence of HPV in said sample is determined according to results of said PCR assays; wherein, preferably, said PCR assays are conducted using a set of primer pairs capable of detecting HPVs of said at least 65 genotypes, wherein one or more primer pairs in said set is(are) degenerate primer pair(s).

3. The method according to claim 2, comprising the following steps:
1) Obtaining DNAs from said sample;
2) Reacting the DNAs obtained in step 1) with each primer pair in said set of primer pairs capable of detecting HPVs of said at least 65 genotypes in a PCR assay separately, wherein, preferably, one or more primer pairs in said set is(are) degenerate primer pair(s); and
3) Determining the presence of HPV in said sample according to results of said PCR assays.

4. The method according to claim 2, wherein said step of determining the presence of HPV in said sample according to results of said PCR assays comprises comparing the measured Ct in each of the PCR assays with a reference Ct; wherein the result of the PCR assay is to be determined positive when said measured Ct is less than said reference Ct or when a unimodal dissociation curve of PCR product is observed and said measured Ct is less than said reference Ct, and the result of the PCR assay is to be determined negative when said measured Ct is greater than or equal to said reference Ct or no logarithmic amplification curve is observed; and whereby, said sample is to be determined positive for presence of HPV when the PCR assay with any one of said primer pairs gives a positive result; wherein, preferably, said reference Ct ranges from 30 to 35, from 32 to 35 or from 33 to 35, or is 34 or 35.

5. The method according to claim 2, wherein said set of primer pairs include one or more primer pairs selected from the following list, wherein F refers to the forward primer and R refers to the reverse primer as paired:
| | |
|---|---|
| F1: CWGGWRCWGAYAATAGRGAAAATG, | R1: YRCCATATCMCCATCCTGWATA; |
| F2: GCRACAAWCTTGAGGACCARAT, | R2: YGTGTGCTAGATACRGGTTCAAATG; |
| F3: TGCGTGCCAGGAGABGYTAATAGAC, | R3: RKGTCCTGGGTRCTAGATAC; |
| F4: CCYGAKGTGRTTGACCTACA, | R4: VVHRTYRGCCATGTCTCTTATG; |
| F5: TGCWGAGCCBTATGGCGATTG, | R5: ACMCCAAAATTCCACTCATC; |
| F6: TGTGGCCTAAACGACGTAAAC, | R6: CGCGTGACATAGACATCCGTACTG; |
| F7: GGAGAMGTTRCTAGAACTGTATGA, | R7: GGYDCTGYGTCCCACATTTC; |
| F8: GTGCCAGGAGAAAATACTAGACT, | R8: YSCYGBAGGTACTAGATACA; |
| F9: YGCCTATGGBGATTCTATGTG, | R9: CCACTAGGHGTAGCAACATATAC; |
| F10: GGCMCAGGGYCATAACAATGGT, | R10: DCGWCCAAGRGGATATTGAT; |
| F11: GCWGATGTRTATGGRGACAGTATGT, | R11: RGGCTTRTTAAAYAACTGGGARTC; |
| F12: TGTCCACCAATGCTTATTACA, | R12: CCTCTTCCTSGTKCAAATCTAATC; |
| F13: GATGGWAACCCAACTAGYATAG, | R13: CCGTTGCTGTCAAATGGAAATG; |
| F14: ASYGAAGTATCCTCTGTTGCGTCTA, | R14: TCGAATCGCACTCGCTTTCTA; |
| F15: GTTGTWAGCACKGATGAATATG, | R15: CCACTAATRCCYACACCYAATG; |
| F16: GACGTGARCARATGTTTGTTAG, | R16: GGWGGKGTWARRCCAAATTG; |
| F17: TRCACGYARACGCCGTAAAC, | R17: KKCCYACWGCAAGTAGTCTA; and |
| F18: GGCMWTRTTAGATGATGCWACA, | R18: CACGTTCATACAGTTCTAGGA; |
wherein R represents A or G; Y represents C or T; M represents A or C; K represents G or T; S represents C or G; W represents A or T; H represents A, C or T; B represents C, G or T; V represents A, C or G; and D represents A, G or T.

6. The method according to claim 2, wherein,
(a) annealing temperatures for said PCR assays are independently 40°C-60°C, preferably 50°C-60°C, and more preferably 55°C; or/and,
(b) said PCR assays each comprise independently 30 to 50 cycles, preferably 35-45 cycles and more preferably 40 cycles of PCR reaction.

7. The method according to claim 1, wherein, said sample is selected from the group consisting of cells, blood or secretions; or, said sample is a sample of cells, such as stem cells, particularly mesenchymal stem cells.

8. A kit for detection of human papillomavirus, comprising one or more reagents capable of detecting HPVs of at least the following 65 genotypes: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73, and HPV177 wherein said reagents comprise or consist of a set of primer pairs which is capable of detecting the at least 65 HPV genotypes.

9. The kit according to claim 8, wherein one or more primer pairs in said set is(are) degenerate primer pair(s); and preferably, said set of primer pairs include one or more primer pairs selected from the following list, wherein F refers to the forward primer and R refers to the reverse primer as paired:
| | |
|---|---|
| F1: CWGGWRCWGAYAATAGRGAAAATG, | R1: YRCCATATCMCCATCCTGWATA; |
| F2: GCRACAAWCTTGAGGACCARAT, | R2: YGTGTGCTAGATACRGGTTCAAATG; |
| F3: TGCGTGCCAGGAGABGYTAATAGAC, | R3: RKGTCCTGGGTRCTAGATAC; |
| F4: CCYGAKGTGRTTGACCTACA, | R4: VVHRTYRGCCATGTCTCTTATG; |
| F5: TGCWGAGCCBTATGGCGATTG, | R5: ACMCCAAAATTCCACTCATC; |
| F6: TGTGGCCTAAACGACGTAAAC, | R6: CGCGTGACATAGACATCCGTACTG; |
| F7: GGAGAMGTTRCTAGAACTGTATGA, | R7: GGYDCTGYGTCCCACATTTC; |
| F8: GTGCCAGGAGAAAATACTAGACT, | R8: YSCYGBAGGTACTAGATACA; |
| F9: YGCCTATGGBGATTCTATGTG, | R9: CCACTAGGHGTAGCAACATATAC; |
| F10: GGCMCAGGGYCATAACAATGGT, | R10: DCGWCCAAGRGGATATTGAT; |
| F11: GCWGATGTRTATGGRGACAGTATGT, | R11: RGGCTTRTTAAAYAACTGGGARTC; |
| F12: TGTCCACCAATGCTTATTACA, | R12: CCTCTTCCTSGTKCAAATCTAATC; |
| F13: GATGGWAACCCAACTAGYATAG, | R13: CCGTTGCTGTCAAATGGAAATG; |
| F14: ASYGAAGTATCCTCTGTTGCGTCTA, | R14: TCGAATCGCACTCGCTTTCTA; |
| F15: GTTGTWAGCACKGATGAATATG, | R15: CCACTAATRCCYACACCYAATG; |
| F16: GACGTGARCARATGTTTGTTAG, | R16: GGWGGKGTWARRCCAAATTG; |
| F17: TRCACGYARACGCCGTAAAC, | R17: KKCCYACWGCAAGTAGTCTA; and |
| F18: GGCMWTRTTAGATGATGCWACA, | R18: CACGTTCATACAGTTCTAGGA; |
wherein R represents A or G; Y represents C or T; M represents A or C; K represents G or T; S represents C or G; W represents A or T; H represents A, C or T; B represents C, G or T; V represents A, C or G; and D represents A, G or T.

10. The kit according to claim 8, which is **characterized in** one or more of the following features:
(a) Said kit is one for PCR-based assays; preferably, said kit further comprises other components of a polymerase chain reaction system;
(b) Said kit is one for assays on a sample selected from the group consisting of cells, blood or secretions; and/or
(c) Said kit is one for assays on a sample of cells, such as stem cells, particularly mesenchymal stem cells.

11. A set of primer pairs which is capable of detecting the at least 65 HPV genotypes defined in claim 8, comprising one or more primer pairs selected from the following list, wherein F refers to the forward primer and R refers to the reverse primer as paired:
| | |
|---|---|
| F1: CWGGWRCWGAYAATAGRGAAAATG, | R1: YRCCATATCMCCATCCTGWATA; |
| F2: GCRACAAWCTTGAGGACCARAT, | R2: YGTGTGCTAGATACRGGTTCAAATG; |
| F3: TGCGTGCCAGGAGABGYTAATAGAC, | R3: RKGTCCTGGGTRCTAGATAC; |
| F4: CCYGAKGTGRTTGACCTACA, | R4: VVHRTYRGCCATGTCTCTTATG; |
| F5: TGCWGAGCCBTATGGCGATTG, | R5: ACMCCAAAATTCCACTCATC; |
| F6: TGTGGCCTAAACGACGTAAAC, | R6: CGCGTGACATAGACATCCGTACTG; |
| F7: GGAGAMGTTRCTAGAACTGTATGA, | R7: GGYDCTGYGTCCCACATTTC; |
| F8: GTGCCAGGAGAAAATACTAGACT, | R8: YSCYGBAGGTACTAGATACA; |
| F9: YGCCTATGGBGATTCTATGTG, | R9: CCACTAGGHGTAGCAACATATAC; |
| F10: GGCMCAGGGYCATAACAATGGT, | R10: DCGWCCAAGRGGATATTGAT; |
| F11: GCWGATGTRTATGGRGACAGTATGT, | R11: RGGCTTRTTAAAYAACTGGGARTC; |
| F12: TGTCCACCAATGCTTATTACA, | R12: CCTCTTCCTSGTKCAAATCTAATC; |
| F13: GATGGWAACCCAACTAGYATAG, | R13: CCGTTGCTGTCAAATGGAAATG; |
| F14: ASYGAAGTATCCTCTGTTGCGTCTA, | R14: TCGAATCGCACTCGCTTTCTA; |
| F15: GTTGTWAGCACKGATGAATATG, | R15: CCACTAATRCCYACACCYAATG; |
| F16: GACGTGARCARATGTTTGTTAG, | R16: GGWGGKGTWARRCCAAATTG; |
| F17: TRCACGYARACGCCGTAAAC, | R17: KKCCYACWGCAAGTAGTCTA; and |
| F18: GGCMWTRTTAGATGATGCWACA, | R18: CACGTTCATACAGTTCTAGGA; |
wherein R represents A or G; Y represents C or T; M represents A or C; K represents G or T; S represents C or G; W represents A or T; H represents A, C or T; B represents C, G or T; V represents A, C or G; and D represents A, G or T.

## Patentansprüche

1. Verfahren zum Nachweisen des Vorhandenseins von humanem Papillomavirus (HPV) in einer Probe, umfassend den Schritt des Nachweisens des Vorhandenseins von HPVs von mindestens den folgenden 65 Genotypen: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73 und HPV177.

2. Verfahren nach Anspruch 1, wobei das Vorhandensein der Genotypen über Polymerase-Kettenreaktionsassays (PCR-Assays) nachgewiesen wird und das Vorhandensein von HPV in der Probe gemäß Ergebnissen der PCR-Assays bestimmt wird; wobei die PCR-Assays vorzugsweise unter Verwendung eines Satzes von Primerpaaren durchgeführt werden, die in der Lage sind, HPVs der mindestens 65 Genotypen nachzuweisen, wobei ein oder mehrere Primerpaare in dem Satz degenerierte Primerpaare sind.

3. Verfahren nach Anspruch 2, umfassend die folgenden Schritte:
1) Erhalten von DNAs aus der Probe;
2) Umsetzen der in Schritt 1) erhaltenen DNAs mit jedem Primerpaar in dem Satz von Primerpaaren, die in der Lage sind, HPVs der mindestens 65 Genotypen in einem PCR-Assay separat nachzuweisen, wobei vorzugsweise ein oder mehrere Primerpaare in dem Satz degenerierte Primerpaare sind; und
3) Bestimmen des Vorhandenseins von HPV in der Probe gemäß Ergebnissen der PCR-Assays.

4. Verfahren nach Anspruch 2, wobei der Schritt des Bestimmens des Vorhandenseins von HPV in der Probe gemäß Ergebnissen der PCR-Assays Vergleichen des gemessenen Ct in jedem der PCR-Assays mit einem Referenz-Ct umfasst; wobei das Ergebnis des PCR-Assays als positiv zu bestimmen ist, wenn der gemessene Ct kleiner als der Referenz-Ct ist oder wenn eine unimodale Dissoziationskurve des PCR-Produkts beobachtet wird und der gemessene Ct kleiner als der Referenz-Ct ist, und das Ergebnis des PCR-Assays als negativ zu bestimmen ist, wenn der gemessene Ct größer oder gleich dem Referenz-Ct ist oder keine logarithmische Amplifikationskurve beobachtet wird; und wobei die Probe als positiv für das Vorhandensein von HPV zu bestimmen ist, wenn der PCR-Assay mit einem beliebigen der Primerpaare ein positives Ergebnis ergibt; wobei der Referenz-Ct vorzugsweise von 30 bis 35, von 32 bis 35 oder von 33 bis 35 reicht oder 34 oder 35 beträgt.

5. Verfahren nach Anspruch 2, wobei der Satz von Primerpaaren ein oder mehrere Primerpaare enthalten, die aus der folgenden Liste ausgewählt sind, wobei sich F auf den Vorwärtsprimer und R auf den Rückwärtsprimer wie gepaart bezieht:
| | |
|---|---|
| F1: CWGGWRCWGAYAATAGRGAAAATG, | R1: YRCCATATCMCCATCCTGWATA; |
| F2: GCRACAAWCTTGAGGACCARAT, | R2: YGTGTGCTAGATACRGGTTCAAATG; |
| F3: TGCGTGCCAGGAGABGYTAATAGAC, | R3: RKGTCCTGGGTRCTAGATAC; |
| F4: CCYGAKGTGRTTGACCTACA, | R4: VVHRTYRGCCATGTCTCTTATG; |
| F5: TGCWGAGCCBTATGGCGATTG, | R5: ACMCCAAAATTCCACTCATC; |
| F6: TGTGGCCTAAACGACGTAAAC, | R6: CGCGTGACATAGACATCCGTACTG; |
| F7: GGAGAMGTTRCTAGAACTGTATGA, | R7: GGYDCTGYGTCCCACATTTC; |
| F8: GTGCCAGGAGAAAATACTAGACT, | R8: YSCYGBAGGTACTAGATACA; |
| F9: YGCCTATGGBGATTCTATGTG, | R9: CCACTAGGHGTAGCAACATATAC; |
| F10: GGCMCAGGGYCATAACAATGGT, | R10: DCGWCCAAGRGGATATTGAT; |
| F11: GCWGATGTRTATGGRGACAGTATGT, | R11: RGGCTTRTTAAAYAACTGGGARTC; |
| F12: TGTCCACCAATGCTTATTACA, | R12: CCTCTTCCTSGTKCAAATCTAATC; |
| F13: GATGGWAACCCAACTAGYATAG, | R13: CCGTTGCTGTCAAATGGAAATG; |
| F14: ASYGAAGTATCCTCTGTTGCGTCTA, | R14: TCGAATCGCACTCGCTTTCTA; |
| F15: GTTGTWAGCACKGATGAATATG, | R15: CCACTAATRCCYACACCYAATG; |
| F16: GACGTGARCARATGTTTGTTAG, | R16: GGWGGKGTWARRCCAAATTG; |
| F17: TRCACGYARACGCCGTAAAC, | R17: KKCCYACWGCAAGTAGTCTA; und |
| F18: GGCMWTRTTAGATGATGCWACA, | R18: CACGTTCATACAGTTCTAGGA; |
wobei R A oder G repräsentiert; Y C oder T repräsentiert; M A oder C repräsentiert; K G oder T repräsentiert; S C oder G repräsentiert; W A oder T repräsentiert; H A, C oder T repräsentiert; B C, G oder T repräsentiert; V A, C oder G repräsentiert; und D A, G oder T repräsentiert.

6. Verfahren nach Anspruch 2, wobei
(a) die Anlagerungstemperaturen für die PCR-Assays unabhängig 40 °C bis 60 °C, vorzugsweise 50 °C bis 60 °C und mehr bevorzugt 55 °C betragen; oder/und
(b) die PCR-Assays jeweils unabhängig 30 bis 50 Zyklen, vorzugsweise 35 bis 45 Zyklen und mehr bevorzugt 40 Zyklen der PCR-Reaktion umfassen.

7. Verfahren nach Anspruch 1, wobei die Probe aus der Gruppe ausgewählt ist, die aus Zellen, Blut oder Sekreten besteht; oder wobei die Probe eine Probe von Zellen ist, wie Stammzellen, insbesondere mesenchymale Stammzellen.

8. Kit zum Nachweis des humanen Papillomavirus, umfassend ein oder mehrere Reagenzien, die in der Lage sind, HPVs von mindestens den folgenden 65 Genotypen nachzuweisen: HPV32, HPV42, HPV54, HPV3, HPV10, HPV28, HPV29, HPV77, HPV78, HPV94, HPV117, HPV125, HPV160, HPV83, HPV102, HPV89, HPV61, HPV62, HPV72, HPV81, HPV84, HPV86, HPV87, HPV114, HPV2, HPV27, HPV57, HPV71, HPV90, HPV106, HPV26, HPV51, HPV69, HPV82, HPV30, HPV53, HPV56, HPV66, HPV18, HPV45, HPV97, HPV39, HPV68, HPV70, HPV59, HPV85, HPV7, HPV40, HPV43, HPV91, HPV16, HPV31, HPV35, HPV33, HPV58, HPV52, HPV67, HPV6, HPV11, HPV13, HPV44, HPV74, HPV34, HPV73 und HPV177, wobei die Reagenzien einen Satz von Primerpaaren umfassen oder daraus bestehen, der in der Lage ist, die mindestens 65 HPV-Genotypen nachzuweisen.

9. Kit nach Anspruch 8, wobei ein oder mehrere Primerpaare in dem Satz degenerierte Primerpaare sind; und wobei der Satz von Primerpaaren vorzugsweise ein oder mehrere Primerpaare enthalten, ausgewählt aus der folgenden Liste, wobei sich F auf den Vorwärtsprimer und R auf den Rückwärtsprimer wie gepaart bezieht:
| | |
|---|---|
| F1: CWGGWRCWGAYAATAGRGAAAATG, | R1: YRCCATATCMCCATCCTGWATA; |
| F2: GCRACAAWCTTGAGGACCARAT, | R2: YGTGTGCTAGATACRGGTTCAAATG; |
| F3: TGCGTGCCAGGAGABGYTAATAGAC, | R3: RKGTCCTGGGTRCTAGATAC; |
| F4: CCYGAKGTGRTTGACCTACA, | R4: VVHRTYRGCCATGTCTCTTATG; |
| F5: TGCWGAGCCBTATGGCGATTG, | R5: ACMCCAAAATTCCACTCATC; |
| F6: TGTGGCCTAAACGACGTAAAC, | R6: CGCGTGACATAGACATCCGTACTG; |
| F7: GGAGAMGTTRCTAGAACTGTATGA, | R7: GGYDCTGYGTCCCACATTTC; |
| F8: GTGCCAGGAGAAAATACTAGACT, | R8: YSCYGBAGGTACTAGATACA; |
| F9: YGCCTATGGBGATTCTATGTG, | R9: CCACTAGGHGTAGCAACATATAC; |
| F10: GGCMCAGGGYCATAACAATGGT, | R10: DCGWCCAAGRGGATATTGAT; |
| F11: GCWGATGTRTATGGRGACAGTATGT, | R11: RGGCTTRTTAAAYAACTGGGARTC; |
| F12: TGTCCACCAATGCTTATTACA, | R12: CCTCTTCCTSGTKCAAATCTAATC; |
| F13: GATGGWAACCCAACTAGYATAG, | R13: CCGTTGCTGTCAAATGGAAATG; |
| F14: ASYGAAGTATCCTCTGTTGCGTCTA, | R14: TCGAATCGCACTCGCTTTCTA; |
| F15: GTTGTWAGCACKGATGAATATG, | R15: CCACTAATRCCYACACCYAATG; |
| F16: GACGTGARCARATGTTTGTTAG, | R16: GGWGGKGTWARRCCAAATTG; |
| F17: TRCACGYARACGCCGTAAAC, | R17: KKCCYACWGCAAGTAGTCTA; und |
| F18: GGCMWTRTTAGATGATGCWACA, | R18: CACGTTCATACAGTTCTAGGA; |
wobei R A oder G repräsentiert; Y C oder T repräsentiert; M A oder C repräsentiert; K G oder T repräsentiert; S C oder G repräsentiert; W A oder T repräsentiert; H A, C oder T repräsentiert; B C, G oder T repräsentiert; V A, C oder G repräsentiert; und D A, G oder T repräsentiert.

10. Kit nach Anspruch 8, das durch eines oder mehrere der folgenden Merkmale gekennzeichnet ist:
(a) das Kit ist eins für PCR-basierte Assays; vorzugsweise umfasst das Kit ferner weitere Komponenten eines Polymerase-Kettenreaktionssystems;
(b) das Kit ist eins für Assays an einer Probe, die aus der Gruppe ausgewählt ist, die aus Zellen, Blut oder Sekreten besteht; und/oder
(c) das Kit ist eins für Assays an einer Probe von Zellen, wie Stammzellen, insbesondere mesenchymale Stammzellen.

11. Satz von Primerpaaren, der in der Lage ist, die mindestens 65 in Anspruch 8 definierten HPV-Genotypen nachzuweisen, umfassend ein oder mehrere Primerpaare, die aus der folgenden Liste ausgewählt sind, wobei sich F auf den Vorwärtsprimer und R auf den Rückwärtsprimer wie gepaart bezieht:
| | |
|---|---|
| F1: CWGGWRCWGAYAATAGRGAAAATG, | R1: YRCCATATCMCCATCCTGWATA; |
| F2: GCRACAAWCTTGAGGACCARAT, | R2: YGTGTGCTAGATACRGGTTCAAATG; |
| F3: TGCGTGCCAGGAGABGYTAATAGAC, | R3: RKGTCCTGGGTRCTAGATAC; |
| F4: CCYGAKGTGRTTGACCTACA, | R4: VVHRTYRGCCATGTCTCTTATG; |
| F5: TGCWGAGCCBTATGGCGATTG, | R5: ACMCCAAAATTCCACTCATC; |
| F6: TGTGGCCTAAACGACGTAAAC, | R6: CGCGTGACATAGACATCCGTACTG; |
| F7: GGAGAMGTTRCTAGAACTGTATGA, | R7: GGYDCTGYGTCCCACATTTC; |
| F8: GTGCCAGGAGAAAATACTAGACT, | R8: YSCYGBAGGTACTAGATACA; |
| F9: YGCCTATGGBGATTCTATGTG, | R9: CCACTAGGHGTAGCAACATATAC; |
| F10: GGCMCAGGGYCATAACAATGGT, | R10: DCGWCCAAGRGGATATTGAT; |
| F11: GCWGATGTRTATGGRGACAGTATGT, | R11: RGGCTTRTTAAAYAACTGGGARTC; |
| F12: TGTCCACCAATGCTTATTACA, | R12: CCTCTTCCTSGTKCAAATCTAATC; |
| F13: GATGGWAACCCAACTAGYATAG, | R13: CCGTTGCTGTCAAATGGAAATG; |
| F14: ASYGAAGTATCCTCTGTTGCGTCTA, | R14: TCGAATCGCACTCGCTTTCTA; |
| F15: GTTGTWAGCACKGATGAATATG, | R15: CCACTAATRCCYACACCYAATG; |
| F16: GACGTGARCARATGTTTGTTAG, | R16: GGWGGKGTWARRCCAAATTG; |
| F17: TRCACGYARACGCCGTAAAC, | R17: KKCCYACWGCAAGTAGTCTA; und |
| F18: GGCMWTRTTAGATGATGCWACA, | R18: CACGTTCATACAGTTCTAGGA; |
wobei R A oder G repräsentiert; Y C oder T repräsentiert; M A oder C repräsentiert; K G oder T repräsentiert; S C oder G repräsentiert; W A oder T repräsentiert; H A, C oder T repräsentiert; B C, G oder T repräsentiert; V A, C oder G repräsentiert; und D A, G oder T repräsentiert.

## Revendications

1. Procédé de détection de la présence du virus du papillome humain (VPH) dans un échantillon, comprenant l'étape de détection de la présence de VPH d'au moins les 65 génotypes suivants : VPH32, VPH42, VPH54, VPH3, VPH10, VPH28, VPH29, VPH77, VPH78, VPH94, VPH117, VPH125, VPH160, VPH83, VPH102, VPH89, VPH61, VPH62, VPH72, VPH81, VPH84, VPH86, VPH87, VPH114, VPH2, VPH27, VPH57, VPH71, VPH90, VPH106, VPH26, VPH51, VPH69, VPH82, VPH30, VPH53, VPH56, VPH66, VPH18, VPH45, VPH97, VPH39, VPH68, VPH70, VPH59, VPH85, VPH7, VPH40, VPH43, VPH91, VPH16, VPH31, VPH35, VPH33, VPH58, VPH52, VPH67, VPH6, VPH11, VPH13, VPH44, VPH74, VPH34, VPH73 et VPH177.

2. Procédé selon la revendication 1, dans lequel la présence desdits génotypes est détectée via des tests de réaction en chaîne par polymérase (RCP) et la présence de VPH dans ledit échantillon est déterminée en fonction des résultats desdits tests RCP ; dans lequel, de préférence, lesdits tests RCP sont effectués en utilisant un ensemble de paires d'amorces capables de détecter les VPH desdits au moins 65 génotypes, dans lequel une ou plusieurs paires d'amorces dans ledit ensemble sont une ou plusieurs paires d'amorces dégénérées.

3. Procédé selon la revendication 2, comprenant les étapes suivantes :
1) Obtention d'ADN à partir dudit échantillon ;
2) Réaction des ADN obtenus à l'étape 1) avec chaque paire d'amorces dudit ensemble de paires d'amorces capable de détecter séparément les VPH desdits au moins 65 génotypes dans un test RCP, dans lequel, de préférence, une ou plusieurs paires d'amorces dudit ensemble sont des paires d'amorces dégénérées ; et
3) Détermination de la présence du VPH dans ledit échantillon en fonction des résultats desdits tests RCP.

4. Procédé selon la revendication 2, dans lequel ladite étape de détermination de la présence du VPH dans ledit échantillon en fonction des résultats des tests RCP comprend la comparaison du Ct mesuré dans chaque test RCP avec un Ct de référence ; le résultat du test RCP étant positif lorsque ledit Ct mesuré est inférieur au Ct de référence ou lorsqu'une courbe de dissociation unimodale du produit RCP est observée et ledit Ct mesuré est inférieur au Ct de référence, et le résultat du test RCP étant négatif lorsque ledit Ct mesuré est supérieur ou égal au Ct de référence ou qu'aucune courbe d'amplification logarithmique n'est observée ; et ledit échantillon étant positif à la présence du VPH lorsque le test RCP avec l'une quelconque desdites paires d'amorces donne un résultat positif ; de préférence, ledit Ct de référence est compris entre 30 et 35, entre 32 et 35 ou entre 33 et 35, ou est égal à 34 ou 35.

5. Procédé selon la revendication 2, dans lequel ledit ensemble de paires d'amorces comprend une ou plusieurs paires d'amorces sélectionnées dans la liste suivante, dans laquelle F désigne l'amorce sens et R désigne l'amorce antisens appariées :
| | | | |
|---|---|---|---|
| F1 : | CWGGWRCWGAYAATAGRGAAAATG, | R1 : | YRCCATATCMCCATCCTGWATA ; |
| F2: | GCRACAAWCTTGAGGACCARAT, | R2: | YGTGTGCTAGATACRGGTTCAAATG; |
| F3: | TGCGTGCCAGGAGABGYTAATAGAC, | R3 : | RKGTCCTGGGTRCTAGATAC ; |
| F4: | CCYGAKGTGRTTGACCTACA, | R4 : | VVHRTYRGCCATGTCTCTTATG ; |
| F5 : | TGCWGAGCCBTATGGCGATTG, | R5 : | ACMCCAAAATTCCACTCATC ; |
| F6 : | TGTGGCCTAAACGACGTAAAC, | R6: | CGCGTGACATAGACATCCGTACTG; |
| F7 : | GGAGAMGTTRCTAGAACTGTATGA, | R7: | GGYDCTGYGTCCCACATTTC; |
| F8 : | GTGCCAGGAGAAAATACTAGACT, | R8: | YSCYGBAGGTACTAGATACA; |
| F9: | YGCCTATGGBGATTCTATGTG, | R9 : | CCACTAGGHGTAGCAACATATAC ; |
| F10 : | GGCMCAGGGYCATAACAATGGT, | R10 : | DCGWCCAAGRGGATATTGAT ; |
| F11 : | GCWGATGTRTATGGRGACAGTATGT, | R11 : | RGGCTTRTTAAAYAACTGGGARTC ; |
| F12 : | TGTCCACCAATGCTTATTACA, | R12 : | CCTCTTCCTSGTKCAAATCTAATC ; |
| F13 : | GATGGWAACCCAACTAGYATAG, | R13 : | CCGTTGCTGTCAAATGGAAATG ; |
| F14 : | ASYGAAGTATCCTCTGTTGCGTCTA, | R14 : | TCGAATCGCACTCGCTTTCTA ; |
| F15 : | GTTGTWAGCACKGATGAATATG, | R15 : | CCACTAATRCCYACACCYAATG ; |
| F16 : | GACGTGARCARATGTTTGTTAG, | R16 : | GGWGGKGTWARRCCAAATTG ; |
| F17 : | TRCACGYARACGCCGTAAAC, | R17 : | KKCCYACWGCAAGTAGTCTA ; et |
| F18 : | GGCMWTRTTAGATGATGCWACA, | R18 : | CACGTTCATACAGTTCTAGGA ; |
dans laquelle R représente A ou G ; Y représente C ou T ; M représente A ou C ; K représente G ou T ; S représente C ou G ; W représente A ou T ; H représente A, C ou T ; B représente C, G ou T ; V représente A, C ou G ; et D représente A, G ou T.

6. Procédé selon la revendication 2, dans lequel,
(a) les températures de recuit pour lesdits tests RCP sont indépendamment de 40 °C à 60 °C, de préférence de 50 °C à 60 °C, et plus préférablement de 55 °C ; ou/et,
(b) lesdits dosages RCP comprennent chacun indépendamment 30 à 50 cycles, de préférence 35 à 45 cycles et plus préférablement 40 cycles de réaction RCP.

7. Procédé selon la revendication 1, dans lequel ledit échantillon est choisi dans le groupe constitué de cellules, de sang ou de sécrétions ; ou ledit échantillon est un échantillon de cellules, telles que des cellules souches, en particulier des cellules souches mésenchymateuses.

8. Un kit de détection du papillomavirus humain, comprenant un ou plusieurs réactifs capables de détecter les VPH d'au moins les 65 génotypes suivants : VPH32, VPH42, VPH54, VPH3, VPH10, VPH28, VPH29, VPH77, VPH78, VPH94, VPH117, VPH125, VPH160, VPH83, VPH102, VPH89, VPH61, VPH62, VPH72, VPH81, VPH84, VPH86, VPH87, VPH114, VPH2, VPH27, VPH57, VPH71, VPH90, VPH106, VPH26, VPH51, VPH69, VPH82, VPH30, VPH53, VPH56, VPH66, VPH18, VPH45, VPH97, VPH39, VPH68, VPH70, VPH59, VPH85, VPH7, VPH40, VPH43, VPH91, VPH16, VPH31, VPH35, VPH33, VPH58, VPH52, VPH67, VPH6, VPH11, VPH13, VPH44, VPH74, VPH34, VPH73 et VPH177, lesdits réactifs comprenant ou étant constitués d'un ensemble de paires d'amorces qui est capable de détecter les au moins 65 génotypes VPH.

9. Kit selon la revendication 8, dans lequel une ou plusieurs paires d'amorces dudit ensemble sont des paires d'amorces dégénérées ; et de préférence, ledit ensemble de paires d'amorces comprend une ou plusieurs paires d'amorces sélectionnées dans la liste suivante, où F désigne l'amorce sens et R désigne l'amorce antisens appariées :
| | | | |
|---|---|---|---|
| F1 : | CWGGWRCWGAYAATAGRGAAAATG, | R1 : | YRCCATATCMCCATCCTGWATA ; |
| F2 : | GCRACAAWCTTGAGGACCARAT, | R2: | YGTGTGCTAGATACRGGTTCAAATG; |
| F3: | TGCGTGCCAGGAGABGYTAATAGAC, | R3 : | RKGTCCTGGGTRCTAGATAC ; |
| F4: | CCYGAKGTGRTTGACCTACA, | R4 : | VVHRTYRGCCATGTCTCTTATG ; |
| F5 : | TGCWGAGCCBTATGGCGATTG, | R5 : | ACMCCAAAATTCCACTCATC ; |
| F6 : | TGTGGCCTAAACGACGTAAAC, | R6: | CGCGTGACATAGACATCCGTACTG; |
| F7 : | GGAGAMGTTRCTAGAACTGTATGA, | R7: | GGYDCTGYGTCCCACATTTC; |
| F8 : | GTGCCAGGAGAAAATACTAGACT, | R8: | YSCYGBAGGTACTAGATACA; |
| F9: | YGCCTATGGBGATTCTATGTG, | R9 : | CCACTAGGHGTAGCAACATATAC ; |
| F10 : | GGCMCAGGGYCATAACAATGGT, | R10 : | DCGWCCAAGRGGATATTGAT ; |
| F11 : | GCWGATGTRTATGGRGACAGTATGT, | R11 : | RGGCTTRTTAAAYAACTGGGARTC ; |
| F12 : | TGTCCACCAATGCTTATTACA, | R12 : | CCTCTTCCTSGTKCAAATCTAATC ; |
| F13 : | GATGGWAACCCAACTAGYATAG, | R13 : | CCGTTGCTGTCAAATGGAAATG ; |
| F14 : | ASYGAAGTATCCTCTGTTGCGTCTA, | R14 : | TCGAATCGCACTCGCTTTCTA ; |
| F15 : | GTTGTWAGCACKGATGAATATG, | R15: | CCACTAATRCCYACACCYAATG; |
| F16 : | GACGTGARCARATGTTTGTTAG, | R16 : | GGWGGKGTWARRCCAAATTG ; |
| F17 : | TRCACGYARACGCCGTAAAC, | R17 : | KKCCYACWGCAAGTAGTCTA ; et |
| F18 : | GGCMWTRTTAGATGATGCWACA, | R18: | CACGTTCATACAGTTCTAGGA; |
dans laquelle R représente A ou G ; Y représente C ou T ; M représente A ou C ; K représente G ou T ; S représente C ou G ; W représente A ou T ; H représente A, C ou T ; B représente C, G ou T ; V représente A, C ou G ; et D représente A, G ou T.

10. Kit selon la revendication 8, **caractérisé par** une ou plusieurs des caractéristiques suivantes :
(a) Ledit kit est destiné aux analyses basées sur la RCP ; de préférence, ledit kit comprend en outre d'autres composants d'un système de réaction en chaîne par polymérase ;
(b) Ledit kit est destiné à des analyses sur un échantillon sélectionné dans le groupe constitué de cellules, de sang ou de sécrétions ; et/ou
(c) Ledit kit est destiné à des dosages sur un échantillon de cellules, telles que des cellules souches, en particulier des cellules souches mésenchymateuses.

11. Ensemble de paires d'amorces qui est capable de détecter les au moins 65 génotypes VPH définis dans la revendication 8, comprenant une ou plusieurs paires d'amorces sélectionnées dans la liste suivante, dans laquelle F désigne l'amorce directe et R désigne l'amorce inverse appariées :
| | | | |
|---|---|---|---|
| F1 : | CWGGWRCWGAYAATAGRGAAAATG, | R1 : | YRCCATATCMCCATCCTGWATA ; |
| F2 : | GCRACAAWCTTGAGGACCARAT, | R2: | YGTGTGCTAGATACRGGTTCAAATG; |
| F3: | TGCGTGCCAGGAGABGYTAATAGAC, | R3: | RKGTCCTGGGTRCTAGATAC ; |
| F4: | CCYGAKGTGRTTGACCTACA, | R4 : | VVHRTYRGCCATGTCTCTTATG ; |
| F5 : | TGCWGAGCCBTATGGCGATTG, | R5 : | ACMCCAAAATTCCACTCATC ; |
| F6 : | TGTGGCCTAAACGACGTAAAC, | R6: | CGCGTGACATAGACATCCGTACTG; |
| F7 : | GGAGAMGTTRCTAGAACTGTATGA, | R7: | GGYDCTGYGTCCCACATTTC; |
| F8 : | GTGCCAGGAGAAAATACTAGACT, | R8: | YSCYGBAGGTACTAGATACA; |
| F9: | YGCCTATGGBGATTCTATGTG, | R9 : | CCACTAGGHGTAGCAACATATAC ; |
| F10 | : GGCMCAGGGYCATAACAATGGT, | R10 : | DCGWCCAAGRGGATATTGAT ; |
| F11 | : GCWGATGTRTATGGRGACAGTATGT, | R11 : | RGGCTTRTTAAAYAACTGGGARTC ; |
| F12 : | TGTCCACCAATGCTTATTACA, | R12 : | CCTCTTCCTSGTKCAAATCTAATC ; |
| F13 : | GATGGWAACCCAACTAGYATAG, | R13 : | CCGTTGCTGTCAAATGGAAATG ; |
| F14 : | ASYGAAGTATCCTCTGTTGCGTCTA, | R14 : | TCGAATCGCACTCGCTTTCTA ; |
| F15 : | GTTGTWAGCACKGATGAATATG, | R15 : | CCACTAATRCCYACACCYAATG ; |
| F16 : | GACGTGARCARATGTTTGTTAG, | R16 : | GGWGGKGTWARRCCAAATTG ; |
| F17 : | TRCACGYARACGCCGTAAAC, | R17 : | KKCCYACWGCAAGTAGTCTA ; et |
| F18 : | GGCMWTRTTAGATGATGCWACA, | R18 : | CACGTTCATACAGTTCTAGGA ; |
dans laquelle R représente A ou G ; Y représente C ou T ; M représente A ou C ; K représente G ou T ; S représente C ou G ; W représente A ou T ; H représente A, C ou T ; B représente C, G ou T ; V représente A, C ou G ; et D représente A, G ou T.
